(19) Europäisches Patentamt
European Patent Office
Office européen des brevets

(11) Numéro de publication: 0 303 871 B1

# (12) FASCICULE DE BREVET EUROPEEN

(45) Date de publication de fascicule du brevet: **09.12.92**

(51) Int. Cl.5: **C07D 498/04**, C07D 239/50, A61K 31/505, A61K 7/06

(21) Numéro de dépôt: **88112300.4**

(22) Date de dépôt: **29.07.88**

## (54) Oxadiazolo pyrimidinones, leur préparation et leur utilisation en cosmétique et dermopharmacie.

(30) Priorité: **31.07.87 LU 86959**

(43) Date de publication de la demande:
**22.02.89 Bulletin 89/08**

(45) Mention de la délivrance du brevet:
**09.12.92 Bulletin 92/50**

(84) Etats contractants désignés:
**AT BE CH DE ES FR GB IT LI NL SE**

(56) Documents cités:
**EP-A- 0 042 669**
**EP-A- 0 054 866**
**WO-A-85/04577**
**WO-A-86/00616**

**HELVETICA CHEMICA ACTA, vol. 66, no. 61, fasc. 2, 1983, pages 669-672, Bâle, CH; U. HENGARTNER et al.: "61. Synthesis of 2-oxo-2H-[1,2,4]oxadiazolo-[2,3-c]pyrimidine--5-carbamates"**

(73) Titulaire: **L'OREAL**
**14, Rue Royale**
**F-75008 Paris(FR)**

(72) Inventeur: **Maignan, Jean**
**8, Rue Halévy**
**F-93290 Tremblay les Gonesse(FR)**
Inventeur: **Restle, Serge**
**140 Rue Anatole France**
**F-93601 Aulnay sous Bois(FR)**
Inventeur: **Lang, Gérard**
**44, Avenue Lacour**
**F-95210 Saint Gratien(FR)**

(74) Mandataire: **Casalonga, Axel et al**
**BUREAU D.A. CASALONGA - JOSSE Morassistrasse 8**
**W-8000 München 5(DE)**

EP 0 303 871 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention est relative à de nouvelles oxadiazolo pyrimidinones, leur préparation et, à des compositions cosmétiques ou pharmaceutiques destinées notamment à être utilisées en application topique.

On connaît déjà, dans l'état de la technique, des oxadiazolo [2,3a] pyrimidinones dont la fonction amine est bloquée sous forme de carbamate, dans les brevets U.S. 4 316 901 et U.S. 4 393 065. Ces produits ont une application dans le domaine de l'élevage pour l'augmentation de la production de viande.

On connaît également des oxadiazolo [2,3c] pyrimidinones, telles que décrites dans le brevet U.S. 4 360 521, pour leurs propriétés vasodilatatrice et anti-hypertenseur. La synthèse de ces produits dérivés de tétrahydro-1,2,3,6 pyridine, à partir de mono ou dicarbamates est décrite dans deux publications de J.C. Muller et coll. Helvetica Chimica Acta - vol. 65 - fasc. 5 (1982) n° 142 page 1445 et vol. 66 fasc. 2 (1983) n° 61 page 669. Ces composés ainsi que leurs isomères, sont connus pour leur utilisation topique comme agents de repousse des cheveux comme le décrit le brevet WO-A-85/04577 (BAZZANO) et WO-A-86/00616.

Le 7-amino |3,6-dihydro-1(2H)-pyridyl|-5(2H)-oxadiazolo-1,2,4 |2,3c| pyrimidinone-2 est un composé intermédiairede ces carbamates ; il est cité dans la publication HELVETICA CHIMICA ACTA volume 66 fas. 2 (1983) n° 61 p. 670.

La demanderesse vient de découvrir de nouveaux dérivés amino-7(aminosubstitué-5)-2H-oxadiazolo-1,2,4 [2,3a] pyrimidinone-2 et leur forme isomère amino-7 (aminosubstitué-5)-2H- oxadiazolo-1,2,4 [2,3-c] pyrimidinone-2.

Elle a découvert que ces nouvelles oxadiazolo pyrimidinones étaient particulièrement efficaces pour la repousse des cheveux et pouvaient être utilisées notamment dans le traitement des maladies du cuir chevelu telles que la pelade, la chute des cheveux, la dermatite desquamante, l'alopécie.

L'invention a donc pour objet de nouvelles oxadiazolo pyrimidinones.

Un autre objet de l'invention est constitué par leur procédé de préparation.

L'invention concerne également les compositions cosmétiques et/ou pharmaceutiques mettant en oeuvre ces composés.

D'autres objets de l'invention apparaîtront à la lecture de la description et des exemples qui suivent.

Les composés conformes à l'invention sont essentiellement caractérisés par le fait qu'il répondent à la formule générale (I) et sa forme isomère (II) :

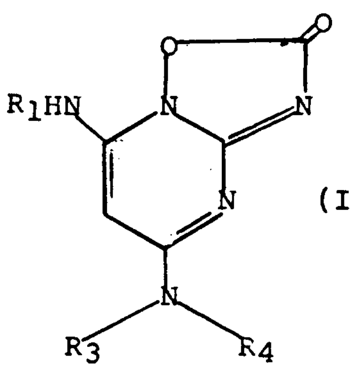
(I)

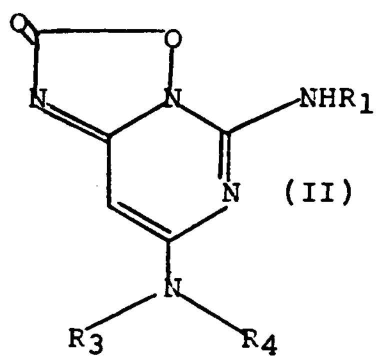
(II)

dans laquelle :

$R_3$ et $R_4$ identiques ou différents, désignent un atome d'hydrogène, un radical alkyle linéaire ou ramifié en $C_1$-$C_{18}$, alcényle ayant 2 à 18 atomes de carbone, cycloalkyle de 5 à 8 atomes de carbone, éventuellement substitué par un ou plusieurs groupements alkyle inférieurs, les groupements alkyle, alcényle ou cycloalkyle pouvant eux-mêmes être substitués par un ou plusieurs groupements hydroxyle,

$R_3$ et/ou $R_4$ désignent également un groupement aryle ou aralkyle répondant à la formule :

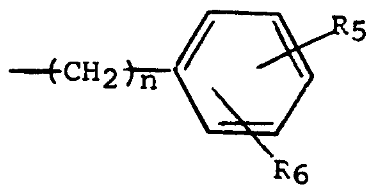

(III)

dans laquelle :

n peut prendre les valeurs de 0 à 4 où $R_5$ et $R_6$, indépendamment l'un de l'autre, désignent hydrogène, un groupement alkyle inférieur en $C_1$-$C_6$, un groupement nitro, hydroxyle, alcoxy ou un atome d'halogène, ou encore un groupement carboxylique ainsi que ses formes salifiées, esters et amides,

$R_3$ et $R_4$ pris ensemble avec l'atome d'azote auquel ils sont reliés, peuvent former un hétérocycle de 3 à 7 atomes de carbone,

$R_1$ désigne un atome d'hydrogène ou un groupement carbamoyle de formule :

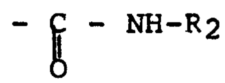

dans laquelle $R_2$ désigne un atome d'hydrogène, un radical alkyle linéaire ou ramifié en $C_1$-$C_{18}$, un groupement alcényle en $C_2$-$C_{18}$, un groupement cycloalkyle en $C_5$-$C_8$,

$R_2$ peut également désigner un radical aryle ou aralkyle répondant à la formule (III) ci-dessus.

Conformément à l'invention, les radicaux alkyle ayant 1 à 18 atomes de carbone sont de préférence choisis parmi les radicaux méthyle, éthyle, propyle, éthyl-2 hexyle, octyle, dodécyle, hexadécyle et octadécyle.

On entend par radical alkyle inférieur un groupement ayant de 1 à 6 atomes de carbone et notamment les groupements méthyle, éthyle, isopropryle, butyle et tertiobutyle.

Les groupements alcényle préférés sont plus particulièrement les groupements allyle, butényle, hexényle, dodécényle, hexadécényle et octadécényle.

Les radicaux aryle ou aralkyle préférés sont plus particulièrement les radicaux phényle, tolyl-4, nitro-2 phényle, nitro-4 phényle, fluoro-4 phényle, chloro-4 phényle, carboxy-2 phényle, carboxy-4 phényle, hydroxy-4 phényle, benzyle, phénéthyle.

Par un groupement hétérocyclique, on entend de préférence les groupements morpholino, pipéridino, pyrrolidino, pipérazino, N-alkyl-4′ pipérazino, dans lequel le groupement alkyle en position 4′ contient de préférence 1 à 6 atomes de carbone dont l'un est éventuellement substitué par un groupement hydroxyle.

Les composés préférés, conformes à la présente invention, sont ceux dans lesquels $R_1$ désigne l'hydrogène et répondant à la formule générale (Ia) ou sa forme isomère (IIa) :

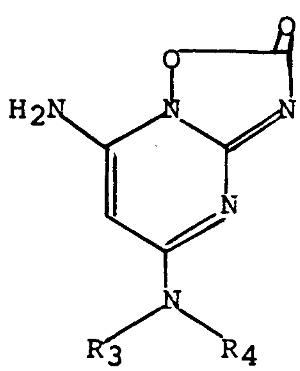

(Ia)

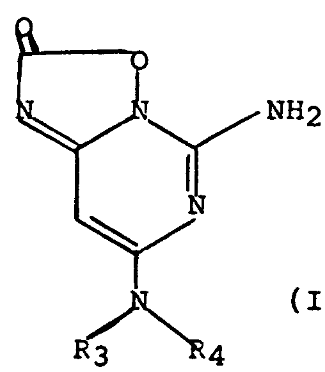

(IIa)

dans lesquelles $R_3$ et $R_4$ ont les mêmes significations indiquées ci-dessus.

D'autres composés préférés conformes à l'invention sont ceux dans lesquels $R_1$ désigne le radical :

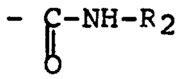

et répondant aux formules (Ib) et (IIb) suivantes :

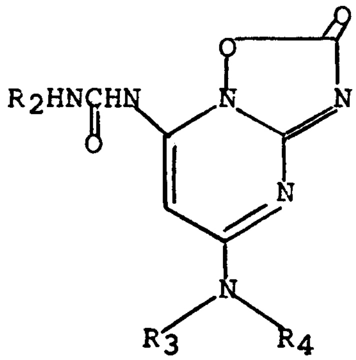

(Ib)

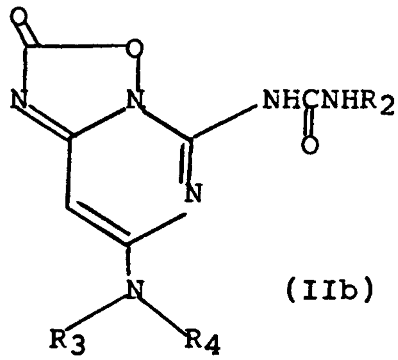

(IIb)

dans lesquelles $R_2$ a les mêmes significations indiquées ci-dessus.

Les composés particulièrement préférés sont ceux dans lesquels $R_3$ et $R_4$ forment avec l'atome d'azote auquel ils sont reliés, un groupement pipéridino et $R_1$ désigne le radical carbamoyle :

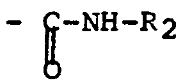

dans lequel $R_2$ est un groupement alkyle inférieur, tel que butyle ou isopropyle, cycloalkyle en $C_5$-$C_8$, en particulier cyclohexyle.

Ces composés répondent à la formule générale (Ic) et sa forme isomère (IIc).

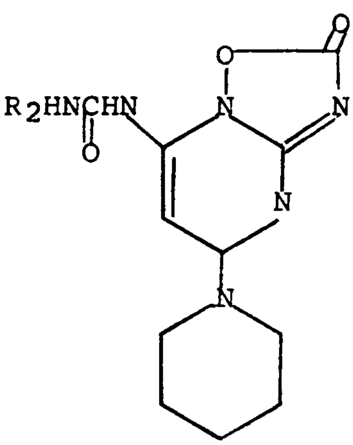

(Ic)

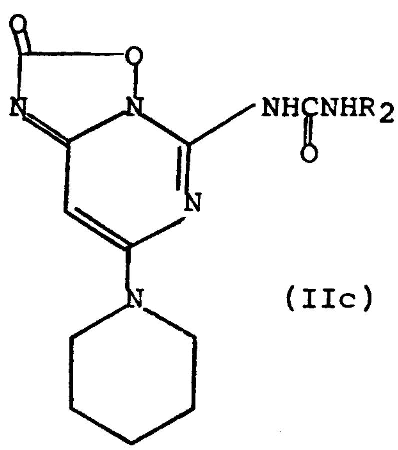

(IIc)

Les composés conformes à la présente invention et en particulier les composés de formule (Ia) et leurs isomères de formule (IIa) sont préparés dans une première variante, selon un procédé qui consiste essentiellement à traiter les monourées de triamino-2,4,6 pyrimidine oxyde-3 dont la fonction en position 6 est disubstituée, de formule générale (VIIa) et leurs isomères de formule (VIIb) :

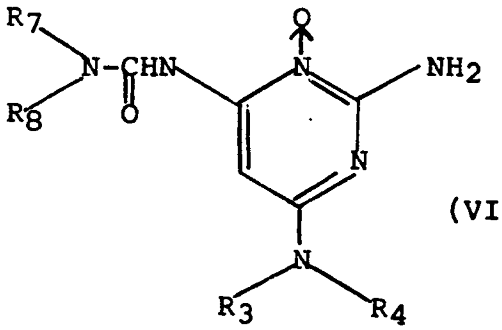

(VIIa)

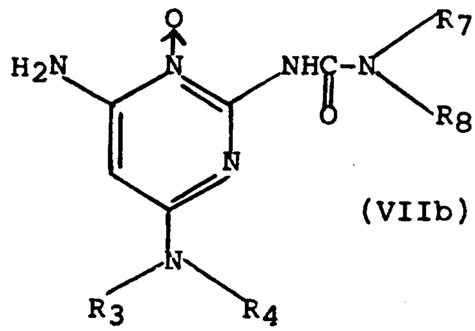

(VIIb)

dans lesquelles $R_3$ et $R_4$ ont les mêmes significations définies dans la formule (I) ci-dessus, et $R_7$ et $R_8$ désignent, indépendamment l'un de l'autre, un radical alkyle linéaire ou ramifié en $C_1$-$C_{18}$, un groupement alcényle en $C_2$-$C_{18}$, un groupement cycloalkyle en $C_5$-$C_8$, un groupement aryle ou aralkyle répondant à la formule (III) définie ci-dessus, en procédant à une réaction d'élimination-cyclisation en présence d'un solvant organique.

Ces composés sont préparés selon un procédé qui consiste à traiter une triamino-2,4,6 pyrimidine oxyde-3 de formule (IV) :

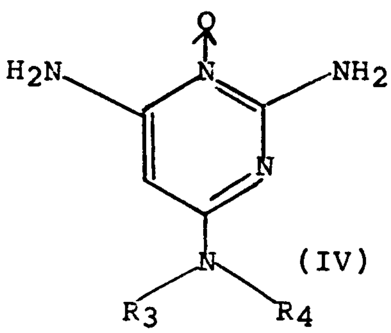

(IV)

dans laquelle $R_3$ et $R_4$ ont les significations définies ci-dessus, en présence d'un solvant polaire aprotique, par un chlorure de carbamoyle de formule (V) :

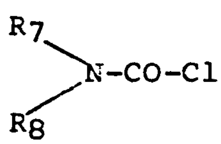

(V)

dans laquelle $R_7$ et $R_8$ ont les mêmes significations définies ci-dessus, puis à traiter, par un équivalent de base forte, le chlorure de N,N-($R_7$,$R_8$) carbamoyloxy-3 triamino-2,4,6 pyrimidinium de formule (VI) :

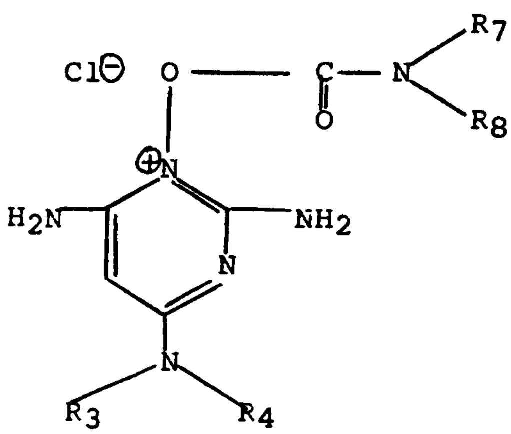

(VI)

dans laquelle $R_3$, $R_4$, $R_7$, $R_8$ ont les significations définies ci-dessus.

Le procédé de préparation des composés de formule (I) conforme à l'invention peut être illustré par le schéma réactionnel A suivant :

SCHEMA REACTIONNEL A

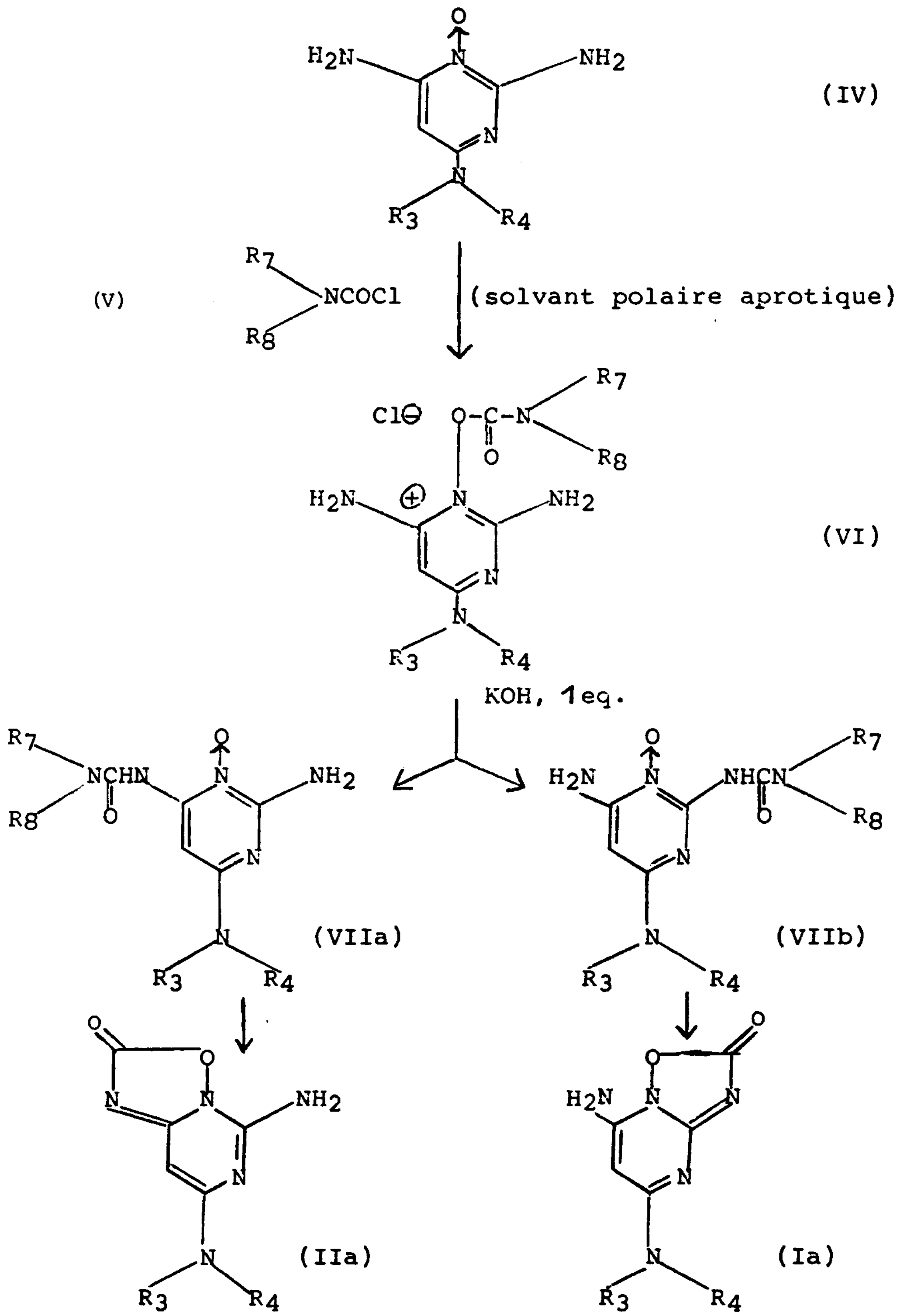


(IV)

(V)

(VI)

(VIIa)

(VIIb)

(IIa)

(Ia)

SCHEMA REACTIONNEL A

La première étape du procédé consiste à faire réagir le chlorure de carbamoyle (V) sur la fonction N-oxyde pour obtenir le sel de formule (VI). Cette réaction est conduite dans un solvant polaire aprotique tel que le dioxanne, le tétrahydrofuranne (THF) ou le diméthylsulfoxyde (DMSO).

Les chlorures de carbamoyle utilisés sont connus en eux-mêmes: certains d'entre eux sont commerciaux, comme par exemple le chlorure de diméthylcarbamoyle ou le chlorure de diisopropylcarbamoyle.

Les sels de structure (VI) préparés dans le dioxanne ou le THF, sont isolés du mélange réactionnel, directement par filtration puis lavage à l'acétone et séchage.

Lorsque la réaction est effectuée dans le DMSO, le mélange réactionnel est directement versé dans l'eau glacée et le sel de formule (VI) précipite ; il est essoré, lavé à l'acétone puis séché.

La seconde étape consiste à traiter les sels obtenus de formule (VI) par un équivalent de base forte telle que la soude ou la potasse pour obtenir le mélange des isomères monourées de formule (VIIa) et (VIIb).

Le composé de formule (VIIa) est prépondérant par rapport à son isomère (VIIb) qui est moins stable.

Dans les conditions d'extraction, d'évaporation des solvants ou de séchage appropriés, le composé isomère (VIIb) conduit d'une manière surprenante, par une réaction d'élimination - cyclisation, à l'oxadiazolo [2,3a] pyrimidinone de formule (Ia).

La stabilté du composé (VIIb) dépend de la fonction amino

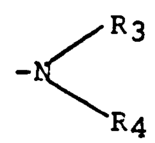

en position 6.

Si l'on souhaite isoler directement les urées de formule (VIIa) et (VIIb) ou l'urée (VIIa) et le produit de cyclisation obtenu à partir de son isomère (VIIb), il est préférable d'utiliser le DMSO dans la première étape et de verser le mélange réactionnel dans l'eau glacée contenant un équivalent de base forte par rapport au chlorure de carbamoyle utilisé.

Les urées (VIIa) et (VIIb) ayant précipité, sont isolées par filtration puis purifiées selon les techniques traditionnelles : chromatographie, cristallisation.

Lorsque l'urée (VIIb) est instable, on sépare son dérivé bi-cyclique (Ia) de l'urée (VIIa).

Lorsque l'urée de formule (VIIb) est stable dans les conditions de purification, on obtient d'une manière quantitative, le composé de formule (Ia) conforme à l'invention, en portant l'urée (VIIb) à une température comprise entre 40 et 100 ° C, pendant quelques heures, dans un solvant organique tel que le toluène.

La forme isomère de structure (IIa) conforme à l'invention est obtenue en portant l'urée de formule (VIIa) dans un solvant organique tel que le toluène ou le xylène, à une température supérieure à 100 ° C pendant quelques heures.

Le rendement est quantitatif et la réaction peut se produire en phase hétérogène.

Lorsqu'on traite les triamino-2,4,6 pyrimidine oxyde-3 de formule (IV) par deux équivalents de chlorure de carbamoyle ou la monourée de formule (VIIa) par un équivalent de chlorure de carbamoyle, on obtient le composé de formule générale (VIIIa), qui correspond à une double acylation de la fonction amine :

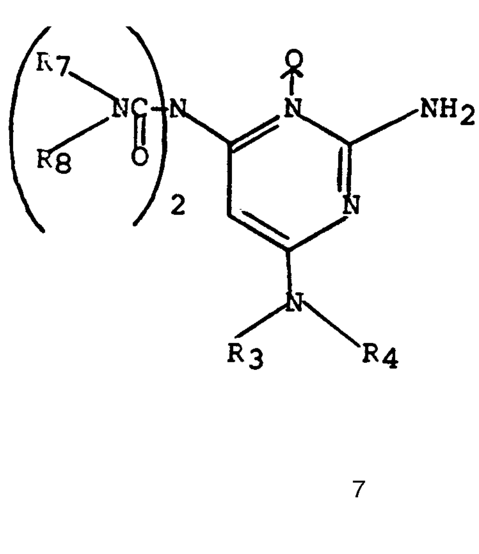

(VIIIa)

dans lesquelles $R_3$ et $R_4$ et $R_7$ et $R_8$ ont les mêmes significations indiquées ci-dessus.

Les composés conformes à l'invention répondant aux formule (Ib) et (IIb) et, notamment les composés particulièrement préférés de formules (Ic) et (IIc), sont obtenus selon différents procédés de préparation.

Un procédé de préparation conforme à l'invention, comme illustré dans le schéma B réactionnel suivant, consiste à traiter l'urée de formule (VIIb) ou son isomère de formule (VIIa), par un isocyanate de formule (IX) :

$R_2 - N = C = O$ (IX)

dans laquelle $R_2$ a la même signification définie ci-dessus dans la formule (I), dans le diméthylsulfoxyde (DMSO).

En versant le milieu réactionnel dans l'eau, l'oxadiazolo pyrimidinone de structure (Ib) précipite. Elle est ensuite essorée et purifiée suivant les techniques classiques.

SCHEMA B

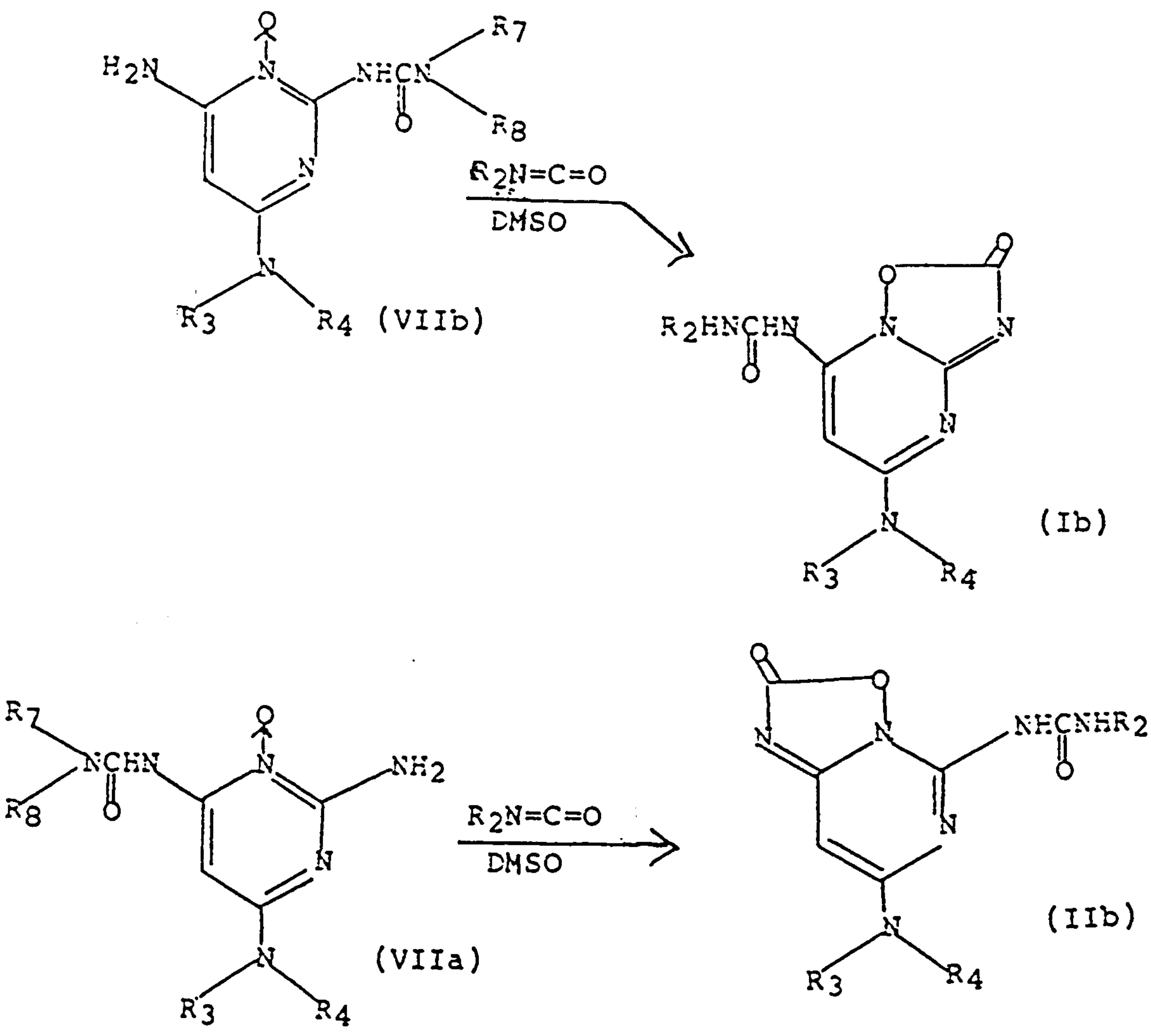


(VIIb) (Ib) (VIIa) (IIb)

Un mode préférentiel conforme à l'invention consiste à traiter directement l'oxadiazolo pyrimidinone de structure (Ia) ou sa forme isomère de structure (IIa) par l'isocyanate $R_2N=C=O$, dans un solvant polaire aprotique tel que le DMSO. Ce procédé peut être illustré par le schéma C réactionnel suivant :

SCHEMA C

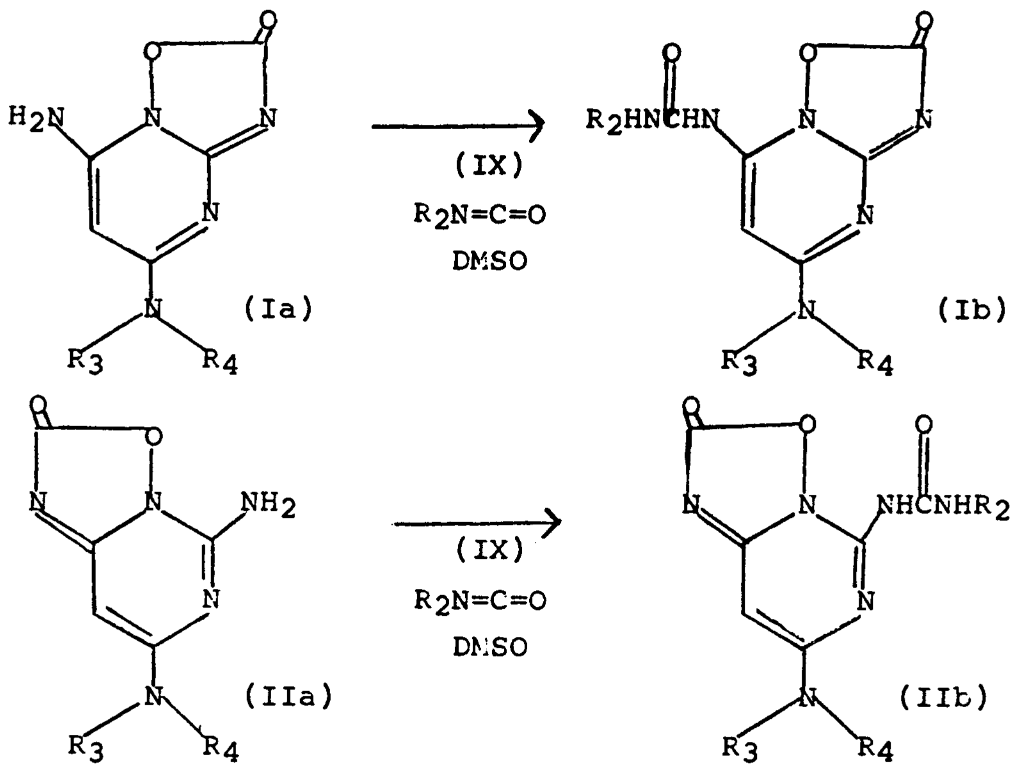


(Ia) (Ib)

(IIa) (IIb)

Un autre procédé de préparation, comme illustré dans le schéma réactionnel D ci-après, conforme à l'invention, consiste à traiter une monourée de formule (Xa) suivante :

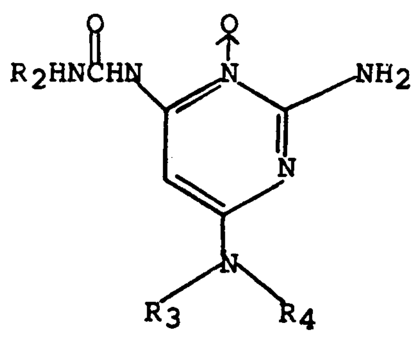

(Xa)

dans laquelle $R_2$, $R_3$ et $R_4$ ont les significations indiquées ci-dessus,

dans le dioxanne, par un chlorure de carbamoyle de formule (V) :

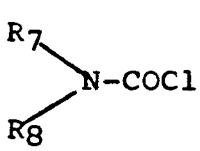 , (V)

pour obtenir la diurée de formule (XIa) :

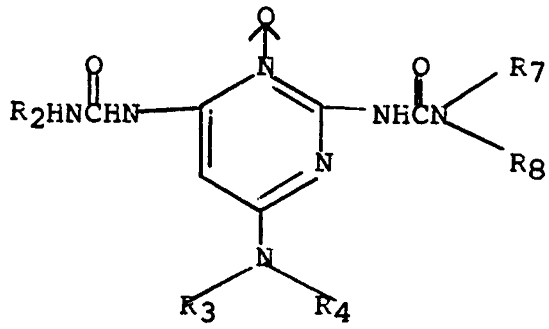

(XIa)

La diurée (XIa) ainsi obtenue, instable est portée, pendant quelques heures, à une température comprise entre 50 et 120 ° C dans un solvant organique tel que le toluène et conduit à l'oxadiazolo pyrimidinone de structure (Ib).

Les composés monourées de formule (Xa) ainsi que leur procédé de préparation, ont fait l'objet d'une demande de brevet au Luxembourg n° 86959.

Selon cette demande, ils sont obtenus, dans une première étape, en traitant la chloro-6 diamino-2,4 pyrimidine par un isocyanate $R_2$-N=C=O, tel que défini ci-dessus, dans un solvant organique polaire aprotique, en procédant, dans une seconde étape, à l'oxydation des dérivés en résultant et en faisant suivre d'un traitement par une amine de formule :

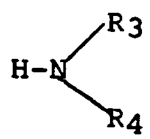

dans laquelle $R_3$ et $R_4$ ont les significations indiquées ci-dessus, pour déplacer l'atome de chlore.

La première étape est mise en oeuvre dans un solvant organique aprotique polaire, tel que par exemple le diméthylsulfoxyde (DMSO), le diméthyl-formamide (DMF), ou la N-méthylpyrrolidone.

L'étape d'oxydation du dérivé en résultant est mise en oeuvre par action d'acide métachloroperbenzoïque ou en utilisant des peracides formés "in situ" par action de l'eau oxygénée sur un acide organique.

On obtient particulièrement de bons résultats en opérant en phase hétérogène dans un mélange d'un solvant chloré tel que le dichlorométhane, ou un éther tel que le dioxanne, en présence d'environ 5% à 30% d'acide formique et de préférence, 10% contenant le composé résultant de la première étape, auquel on ajoute un léger excès d'eau oxygénée. La réaction d'oxydation est conduite de préférence à une température comprise entre 0 et 70 ° C.

Le produit résultant de l'oxydation est isolé avec un très bon rendement, soit en éliminant le solvant chloré par évaporation sous vide, le mélange obtenu est versé dans l'eau et le produit précipité est isolé par filtration, soit en fonction de la nature du groupement $R_2$, directement isolé par filtration du milieu réactionnel.

Les produits obtenus après la réaction d'oxydation sont traités directement par une amine de formule :

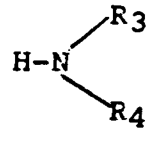

Cette réaction de déplacement du chlore est réalisée suivant la basicité de l'amine :

- soit en utilisant celle-ci en excès; dans ce cas, elle sert à la fois de solvant et de réactif;
- soit en utilisant un solvant organique et l'amine est utilisée en léger excès de 1,5 à 5 équivalents.

Cette réaction est réalisée à une température compatible avec le point d'ébullition de l'amine et est comprise entre 0 et 150 ° C et de préférence aux environs de 70 ° C.

On obtient ainsi, avec un bon rendement, les pyrimidines oxyde de formule (Xa).

En prolongeant le temps de réaction, dans la première étape du procédé, en présence d'un excès d'isocyanate, on peut obtenir le produit de formule :

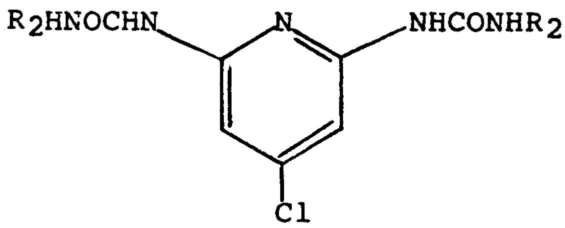

En opérant suivant les deuxième et troisième étapes définies ci-dessus, selon la demande précitée, sur ce composé, on obtient la diurée de formule suivante :

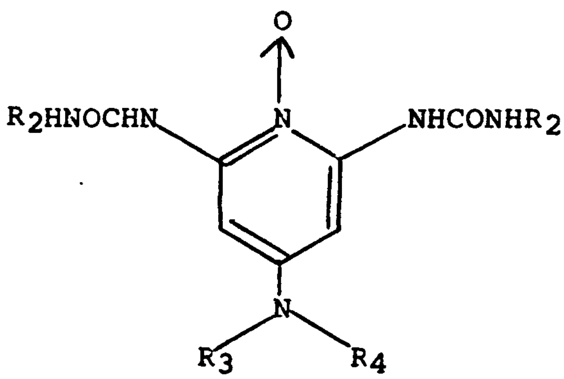

SCHEMA D

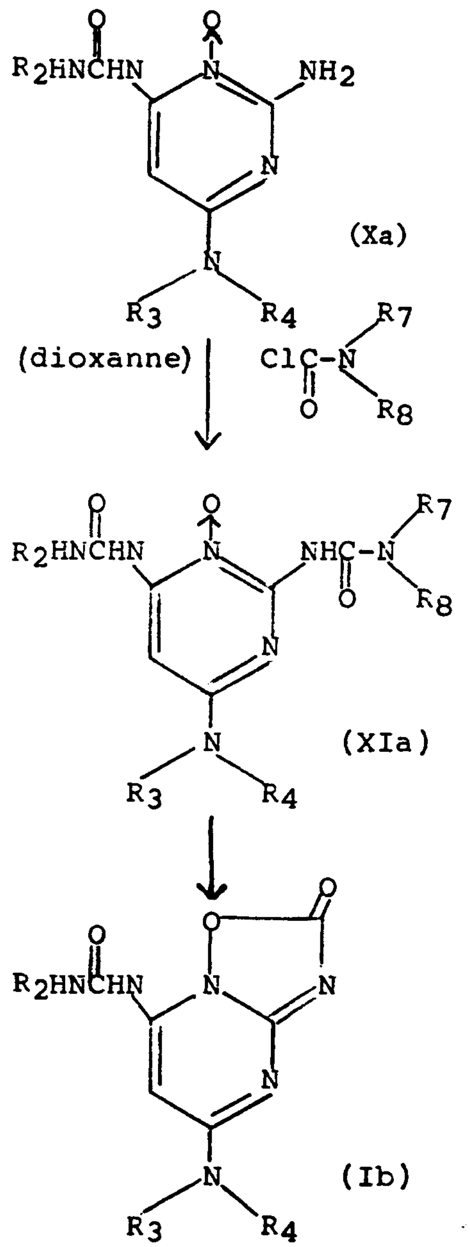


(Xa)

(V)

(XIa)

(Ib)

Les composés conformes à l'invention peuvent être utilisés dans le domaine cosmétique ou pharmaceutique, notamment dans les applications topiques.

La demanderesse a constaté d'une façon surprenante que les composés conformes à l'invention présentent un effet cardiovasculaire nettement inférieur à celui des composés dont la fonction amino en position 2 et/ou 4 est libre. Ils présentent, par ailleurs, une bonne stabilité.

Les compositions cosmétiques ou pharmaceutiques qui constituent un autre objet de l'invention sont utilisées notamment pour traiter le cuir chevelu et plus particulièrement la pelade, la chute des cheveux, l'alopécie, la dermatite desquamante, etc.

Ces compositions sont essentiellement caractérisées par le fait qu'elles contiennent, dans un support pharmaceutiquement ou cosmétiquement approprié pour une application topique, au moins un composé répondant à la formule (I) ou à sa forme isomère (II) et/ou un de ses sels, esters ou amides.

Les compositions conformes à l'invention peuvent comporter tout support approprié pour l'application topique et copatible avec la substance active, les composés pouvant se trouver dans ce support, soit à l'état dissous, soit à l'état dispersé.

Les compositions destinées à être utilisées en pharmacie se présentent sous forme d'onguents, de teintures, de crèmes, de pommades, de poudres, de timbres, de tampons imbibés, de solutions, d'émulsions, de lotions, de gels, de spray ou de suspensions. Elles peuvent être, soit anhydres ou aqueuses selon l'indication clinique.

Les composés sont présents dans ces compositions à des cencentrations comprises entre 0,1 et 10% en poids et en particulier comprises entre 0,2 et 5% en poids.

Les compositions cosmétiques sont notamment destinées à être utilisées sous forme de lotions, de gels, de savons, de mousses ou de shampooings et contiennent dans un support physiologiquement acceptable, au moins un composé de formule (I) ou sa forme isomère de formule (II) ou l'un de ses sels, esters ou amides.

La concentration des composés de formule (I) ou (II) est de préférence comprise dans ces cas là entre 0,01 et 5% en poids, en particulier entre 0,05 et 3% en poids.

Les compositions conformes à l'invention peuvent contenir différents additifs habituellement utilisés en cosmétique ou en pharmacie et qui sont inertes vis-à-vis de la substance active. On peut citer à cet effet des agents hydratants comme la thiamorpholinone et ses dérivés ou l'urée ; des agents antiséborrhéïques tels que la S-carboxyméthylcystéine, la S-benzylcystéamine et leurs dérivés, la tioxolone ; des agents favorisant la repousse des cheveux comme le Phénytoïn (diphényl-5,5 imidazolinedione-2,4) ou encore l'iodure d'oxapropanium, l'acide rétinoïque et ses dérivés, les composés décrits dans les demandes de brevets EP-A 0220118, EP-A 0232199, FR-A 2600064, FR-A 2601002, FR-A 2599031, EP-A 0260162 et GB-A 2197316, l'anthraline et ses dérivés ; des agents anti-inflammatoires stéroïdiens ou non stéroïdiens ; des caroténoïdes et, plus particulièrement, le $\beta$-carotène, les acides eicosatétraynoïque-5,8,11,14 et eicosatriynoïque-5,8,11 et leurs esters et amides.

Les compositions peuvent également contenir des agents conservateurs, des agents stabilisants, des agent régulateurs de pH, des agents modificateurs de pression osmotique, des agents émulsifiants, des filtres UV-A et UV-B, des antioxydants tels que l'$\alpha$-tocophérol, le butylhydroxy-anisole ou le butylhydroxy-toluène.

L'invention a également pour objet un procédé de traitement cosmétique des cheveux consistant à appliquer sur le cuir chevelu au moins une composition telle que définie ci-dessus.

Un autre objet de l'invention est enfin constitué par l'utilisation des composés de formule (I) ou leurs isomère de formule (II) dans la préparation d'un médicament pour le traitement de la pelade, de la chute des cheveux, de la dermatite desquamante.

Les exemples suivants sont destinés à illustrer l'invention sans pour autant présenter un caractère limitatif.

## EXEMPLE I

### Préparation du chlorure de diamino-2,4 (N,N diméthylcarbamoyloxy)-3 pyrrolidino-6 pyrimidinium.

A une suspension agitée sous atmosphère inerte à 50 °C, de 5 g de diamino-2,4 pyrrolidino-6 pyrimidine oxyde-3 dans 40 cm$^3$ de DMSO anhydre, on ajoute goutte à goutte, 2,4 cm$^3$ de chlorure de diméthylcarbamoyle.

Une demi-heure plus tard le mélange réactionnel est homogène. L'agitation est encore maintenue pendant deux heures à température ambiante. Puis la soltuion est versée dans 100 cm$^3$ d'eau et le mélange obtenu abandonné une nuit à température ambiante.

Le lendemain le produit attendu est cristallisé, il est essoré et séché. On obtient 3,5 g de chlorure de diamino-2,4 (N,N-diméthylcarbamoyloxy)-3 pyrrolidino-6 pyrimidinium sous forme de cristaux nacrés blancs de point de fusion de 165 °C.

Ce produit est analysé sous forme d'hydrate : $C_{11}H_{19}Cl\ N_6O_2,\ H_2O$

| | C | H | N | O |
|---|---|---|---|---|
| Calculé | 41,18 | 6,60 | 26,20 | 14,96 |
| Trouvé | 41,16 | 6,51 | 26,55 | 15,20 |

A une solution de 0,4 g de potasse dans 50 cm$^3$ d'éthanol, on ajoute, sous agitation, 2 g de chlorure de diamino-2,4 (N,N-diméthylcarbamoyloxy)-3 pyrrolidino-6 pyrimidinium.

Le milieu est hétérogène et on porte ce mélange pendant deux heures à 60 °C. L'éthanol est éliminé

par évaporation sous vide. Le solide est lavé à l'eau pour éliminer les sels minéraux puis à l'acétone et enfin séché sous pression réduite. On obtient 1,3 g d'un solide dont le spectre $^1H$ RMN montre qu'il est constitué d'un mélange de deux produits dans les proportions 3/2 ; la N-(amino-2 pyrrolidino-6 oxyde-3 pyrimidinyl-4) N′,N′-diméthylurée décrite à l'exemple II et l'amino-7 pyrrolidino-5-2H-oxadiazolo-1,2,4[2,3a] pyrimidinone-2 décrit à l'exemple V.

EXEMPLE II

Préparation de la N-(amino-2 pyrrolidino-6 oxyde-3 pyrimidinyl-4) N′,N′-diméthylurée.

On ajoute goutte à goutte 5,2 $cm^3$ de chlorure de diméthylcarbamoyle à une suspension agitée sous atmosphère inerte à température ambiante, de 10 g de diamino-2,4 pyrrolidino-6 pyrimidine oxyde-3 dans 30 $cm^3$ de diméthylsulfoxyde anhydre. Le mélange devient progressivement homogène.

Deux heures après la fin de l'introduction, la solution est versée dans 100 $cm^3$ d'eau glacée contenant 5,7 $cm^3$ de soude à 30%. Le précipité blanc formé est essoré, puis recristallisé dans le minimum de méthanol.

On obtient 4,6 g de N-(amino-2 pyrrolidino-6 oxyde-3 pyrimidinyl-4) N′,N′-diméthylurée sous forme de cristaux blancs dont le point de fusion est de 231 °C.

| Analyse élémentaire : $C_{11}H_{18}N_6O_2$ | | | | |
|---|---|---|---|---|
| | C | H | N | O |
| Calculé<br>Trouvé | 49,61<br>49,24 | 6,81<br>6,89 | 31,56<br>31,36 | 12,02<br>12,51 |

Son isomère : N-(amino-4 pyrrolidino-6 oxyde-3 pyrimidinyl-2) N′,N′-diméthylurée se forme également au cours de cette réaction; il est instable et n'a pas été purifié.

EXEMPLE III

Préparation de la N-(amino-2 pyrrolidino-6 oxyde-3 pyrimidinyl-4) N′,N′-diisopropylurée et de son is omère N-(amino-4 pyrrolidino-6 oxyde-3 pyrimidinyl-2) N′,N′-diisopropylurée.

A une suspension, agitée sous atmosphère inerte à 50 °C, de 3 g de diamino-2,4 pyrrolidino-6 pyrimidine oxyde-3 dans 10 $cm^3$ de DMSO anhydre, on ajoute par petites quantités 2,8 g de chlorure de diisopropyl-carbamoyle (1,1 équivalent). A la fin de l'addition le mélange est encore agité pendant deux heures à 50 °C, puis à la température ordinaire, il est versé dans 100 $cm^3$ d'eau contenant 2 $cm^3$ de soude 10N. Le précipité jaune formé est essoré, lavé à l'eau et séché. On obtient 3,4 g du mélange d'isomères que l'on fractionne sur une colonne de chromatographie de gel de silice éluée au chlorure de méthylène, puis aux mélanges chlorure de méthylène-méthanol progressivement enrichis en méthanol.

Après évaporation des fractions contenant l'un des isomères on obtient respectivement : - 1,9 g de N-(amino-2 pyrrolidino-6 oxyde-3 pyrimidinyl-4) N′,N′-diisopropylurée sous forme de cristaux blancs fondant à 240 °C.

| Analyse élémentaire : $C_{15}H_{26}N_6O_2$ | | | | |
|---|---|---|---|---|
| | C | H | N | O |
| Calculé<br>Trouvé | 55,88<br>55,74 | 8,13<br>7,98 | 26,07<br>25,74 | 9,92<br>10,26 |

et 0,9 g de N-(amino-4 pyrrolidino-6 oxyde-3 pyrimidinyl-2)N′,N′-diisopropylurée sous forme de cristaux beiges fondant à 263 °C. En fait, ce produit se transforme en dérivé cyclique décrit à l'exemple V. Cette urée est analysée sous forme hydratée :

| $C_{15}H_{26}N_6O_2$, 1/4 $H_2O$ | | | |
|---|---|---|---|
| | C | H | O |
| Calculé<br>Trouvé | 55,11<br>54,37 | 8,17<br>7,80 | 11,01<br>10,91 |

EXEMPLE IV

Préparation de l'amino-7 pyrrolidino-5-2H-oxadiazolo-1,2,4 [2,3-c] pyrimidinone-2.

Une suspension de 500 mg de N-(amino-2 pyrrolidino-6 oxyde-3 pyrimidinyl-4) N′,N′-diisopropylurée dans 30 $cm^3$ de toluène anhydre est portée sous reflux du solvant pendant huit heures, temps au bout duquel le produit de départ est totalement transformé. Ensuite à la température ordinaire le solide est filtré et séché. On obtient 300 mg d'amino-7 pyrrolidino-5-2H-oxadiazolo-1,2,4 [2,3-c] pyrimidinone-2 sous la forme de cristaux blanc-crème de point de fusion 242° C.

| Analyse élémentaire : $C_9H_{11}N_5O_2$ | | | | |
|---|---|---|---|---|
| | C | H | N | O |
| Calculé<br>Trouvé | 48,86<br>48,93 | 5,01<br>5,06 | 31,66<br>31,58 | 14,47<br>14,26 |

EXEMPLE V

Préparation de l'amino-7 pyrrolidino-5-2H-oxadiazolo-1,2,4 [2,3a] pyrimidinone-2.

Une suspension de 300 mg de N-(amino-4 pyrrolidino-6 oxyde-3 pyrimidinyl-2), N′,N′-diisopropylurée dans 30 $cm^3$ de toluène anhydre est portée au reflux du solvant pendant deux heures, temps au bout duquel le produit de départ est totalement transformé. Ensuite, à température ambiante, le solide est essoré puis séché. On obtient 200 mg d'amino-7 pyrrolidino-5-2H-oxadiazolo-1,2,4 [2,3a] pyrimidinone-2 sous forme d'une poudre blanche de point de fusion 263° C.

Le spectre $^1H$ RMN correspond à la structure attendue.

| Analyse élémentaire : $C_9H_{11}N_5O_2$ | | | | |
|---|---|---|---|---|
| | C | H | N | O |
| Calculé<br>Trouvé | 48,86<br>48,44 | 5,01<br>5,00 | 31,66<br>31,40 | 14,47<br>14,85 |

EXEMPLE VI

Préparation du [(N-cyclohexyl carbamoyl) amino]-7 pyrrolidino-5-2H-oxadiazolo-1,2,4 [2,3a] pyrimidinone-2

A une solution de 1 g d'amino-7 pyrrolidino-5-2H-oxadiazolo-1,2,4 [2,3a] pyrimidinone-2 dans 30 $cm^3$ de DMSO anhydre agitée à 60° C sous atmosphère d'azote, on ajoute 0,65 $cm^3$ de cyclohexylisocyanate. Deux heures plus tard, on ajoute de nouveau 0,6 $cm^3$ de cyclohexylisocyanate et le mélange est porté à 80° C pendant six heures, temps au bout duquel la majorité du produit de départ est transformée. Ensuite, le mélange revenu à température ambiante est versé dans 150 $cm^3$ d'eau glacée. Le précipité blanc formé est essoré, lavé à l'eau, séché sous pression réduite puis recristallisé dans l'éthanol. On obtient 0,8 g d [(N-cyclohexylcarbamoyl) amino]-7 pyrrolidino-5-2H-oxadiazolo-1,2,4 [2,3a] pyrimidinone-2 sous forme de cristaux blancs dont le point de fusion est de 244° C. Ce produit est analysé sous forme hydratée.

| Analyse élémentaire : $C_{16}H_{22}N_6O_3$, 1/4 $H_2O$ | | | | |
|---|---|---|---|---|
| | C | H | N | O |
| Calculé<br>Trouvé | 54,76<br>54,83 | 6,46<br>6,48 | 23,95<br>23,88 | 14,82<br>14,58 |

EXEMPLE VII

Préparation du [(N-isopropyl carbamoyl) amino]-7 pyrrolidino-5-2H-oxadiazolo-1,2,4 [2, 3a] pyrimidinone-2

A une solution de 1 g d'amino-7 pyrrolidino-5-2H-oxadiazolo-1,2,4 [2, 3a] pyrimidinone-2 dans 30 $cm^3$ de DMSO anhydre agitée à 80 °C sous atmosphère inerte, on ajoute 0,9 $cm^3$ d'isopropylisocyanate. Le mélange est maintenu sous agitation à 80 °C pendant cinq heures. Le produit de départ étant totalement transformé, la solution est versée dans 150 $cm^3$ d'eau glacée. Le précipité est essoré, lavé à l'eau, séché puis recristallisé dans un mélange acétone-méthanol.

On obtient 0,7 g de [(N-isopropylcarbamoyl) amino]-7 pyrrolidino-5-2H-oxadiazolo-1,2,4 [2, 3a] pyrimidinone-2 sous forme de cristaux blancs dont le point de fusion est de 246 °C.

| Analyse élémentaire : $C_{13}H_{18}N_6O_3$ | | | | |
|---|---|---|---|---|
| | C | H | N | O |
| Calculé<br>Trouvé | 50,97<br>50,82 | 5,92<br>5,98 | 27,44<br>27,41 | 15,67<br>15,89 |

EXEMPLE VIII

Préparation du chlorure de diamino-2,4 (N,N-diméthyl carbamoyloxy)-3 morpholino-6 pyrimidinium.

A une suspension de 2 g de diamino-2,4 morpholino-6 pyrimidine oxyde-3 dans 70 $cm^3$ de dioxanne anhydre agitée sous atmosphère inerte, on ajoute à la température ordinaire 1 $cm^3$ de chlorure de N,N-diméthyl carbamoyle. Ensuite, le mélange réactionnel est porté pendant deux heures sous reflux.

Le produit de départ étant totalement transformé, le solide est essoré, lavé à l'acétone puis séché sous pression réduite. On obtient 1,9 g de chlorure de diamino-2,4 (N,N-diméthylcarbamoyloxy)-3 morpholino-6 pyrimidinium sous forme de cristaux blancs dont le point de fusion est de 200 °C.

Le spectre $^1H$ RMN 80 MHz correspond à la structure attendue.

| Analyse élémentaire : $C_{11}H_{19}ClN_6O_3$ | | | | | |
|---|---|---|---|---|---|
| | C | H | N | O | Cl |
| Calculé<br>Trouvé | 41,44<br>40,98 | 6,00<br>6,07 | 26,37<br>26,04 | 15,06<br>15,85 | 11,12<br>10,89 |

EXEMPLE IX

Préparation de la N-(amino-2 morpholino-6 oxyde-3 pyrimidinyl-4) N',N'-diméthylurée et de son is omère N-(amino-4 morpholino-6 oxyde-3 pyrimidinyl-2) N',N'-diméthylurée.

a) Préparation du chlorure de diamino-2,4 (N,N-diméthylcarbamoyloxy)-3 morpholino-6 pyrimidinium dans le DMSO.

On ajoute goutte à goutte 4,8 $cm^3$ de chlorure de N,N-diméthylcarbamoyle à une suspension de 10 g de diamino-2,4 morpholino-6 pyrimidine oxyde-3 agitée dans 30 $cm^3$ de diméthylsulfoxyde anhydre sous

atmosphère inerte.

La réaction est exothermique et le mélange devient progressivement homogène. Une heure après la fin de l'introduction, le mélange est versé dans 200 $cm^3$ d'éther éthylique refroidi à 0 ° C.

Un produit "pâteux" décante, il est essoré et séché. On obtient 11 g de sel que l'on place dans 250 $cm^3$ d'acétone sous forte agitation.

les cristaux finement divisés sont essorés et séchés. On isole ainsi 10 g de chlorure de diamino-2,4 (N,N-diméthylcarbamoyloxy)-3 morpholino-6 pyrimidinium sous forme de cristaux blancs dont le point de fusion est de 200 ° C. Ce produit cristallise avec une demi molécule de diméthylsulfoxyde.

b) Transformation de ce sel en urées correspondantes.

Dans une solution de 50 $cm^3$ d'éthanol contenant 2,2 $cm^3$ de solution de soude à 30%, on ajoute par petites portions 7 g de chlorure préparé à l'étape a) ci-dessus. Le chlorure de sodium précipité est éliminé par filtration et le filtrat est concentré sous pression réduite, puis déposé sur une colonne de chromatographie gel de silice que l'on élue au chlorure de méthylène puis au mélange chlorure de méthylène-méthanol progressivement enrichi en méthanol.

Après concentration des phases d'élution à une température inférieure à 35 ° C, on isole à partir des premières fractions, 3,5 g de N-(amino-2 morpholino-6 oxyde-3 pyrimidinyl-4) N′,N′-diméthylurée sous forme de cristaux blancs dont le point de fusion est de 243 ° C.

| Analyse élémentaire : $C_{11}H_{18}N_6O_3$ | | | | |
|---|---|---|---|---|
| | C | H | N | O |
| Calculé | 46,80 | 6,43 | 29,77 | 17,00 |
| Trouvé | 46,84 | 6,47 | 29,67 | 17,30 |

Les fractions suivantes contiennent la N-(amino-4 morpholino-6 oxyde-3 pyrimidinyl-2) N′,N′-diméthylurée. Après évaporation de ces phases d'élution on obtient 1 g de ce produit sous forme de cristaux blancs qui se transforme au moment de la fusion en dérivé cyclique correspondant, décrit à l'exemple XI.

Le spectre $^1H$ RMN 80 MHz de cette urée en position 2 correspond à la structure attendue.

## EXEMPLE X

### Préparation de l'amino-7 morpholino-5-2H-oxadiazolo-1,2,4 [2,3-c] pyrimidinone-2.

Une suspension de 0,5 g de N-(amino-2 morpholino-6 oxyde-3 pyrimidinyl-4) N′,N′-diméthylurée dans 30 $cm^3$ de toluène anhydre, est portée sous reflux du solvant pendant douze heures, temps au bout duquel le produit de départ est totalement transformé. Ensuite, à température ambiante, le solide est filtré, lavé au méthanol puis séché. On obtient 0,4 g d'amino-7 morpholino-5-2H-oxadiazolo-1,2,4 [2,3-c] pyrimidinone-2 sous forme de cristaux blancs dont le point de fusion est de 259 ° C.

| Analyse élémentaire : $C_9H_{11}N_5O_3$ | | | | |
|---|---|---|---|---|
| | C | H | N | O |
| Calculé | 45,56 | 4,67 | 29,53 | 20,24 |
| Trouvé | 45,61 | 4,70 | 29,36 | 20,17 |

## EXAMPLE XI

### Préparation de l'amino-7 morpholino-5-2H-oxadiazolo-1,2,4 [2,3a] pyrimidinone-2.

Une suspension de 0,3 g de N-(amino-4 morpholino-6 oxyde-3 pyrimidinyl-2) N′,N′-diméthylurée dans 300 $cm^3$ de toluène anhydre agitée sous atmosphère inerte est portée au reflux pendant deux heures, temps au bout duquel la totalité du produit de départ est transformée. A température ambiante, le solide est essoré, lavé à l'acétone puis séché. On obtient 0,2 g d'amino-7 morpholino-5-2H-oxadiazolo-1,2,4 [2,3a]

pyrimidinone-2 sous forme d'une poudre blanche dont le point de fusion est de 271 ° C.

Le spectre $^1$H RMN 80 MHz correspond à la structure attendue.

EXEMPLE XII

Préparation du chlorure de diamino-2,4 (N,N-diméthylcarbamoyloxy)-3 pipéridino-6 pyridinium.

A une suspension agitée à la température ambiante de 6 g de diamino-2,4 pipéridino-6 pyrimidine oxyde-3 dans 50 cm$^3$ de dioxanne anhydre, on ajoute goutte à goutte 2,9 cm$^3$ de chlorure de diméthylcarbamoyle. L'agitation est encore maintenue deux heures après la fin de l'addition. Le mélange réactionnel est hétérogène. Il est filtré. Le solide est lavé à l'acétone puis à l'éther et, enfin séché sous pression réduite.

On obtient 8,8 g de chlorure de diamino-2,4 (N,N-diméthylcarbamoyloxy)-3 pipéridino-6 pyrimidinium sous forme de cristaux blancs dont le point de fusion est de 155 ° C.

| Analyse élémentaire : $C_{12}H_{21}ClN_6O_2$ | | | | | |
|---|---|---|---|---|---|
| | C | H | Cl | N | O |
| Calculé | 45,49 | 6,68 | 11,19 | 26,53 | 10,10 |
| Trouvé | 44,82 | 6,63 | 10,95 | 26,20 | 10,67 |

EXEMPLE XIII

Préparation de la N-(amino-2 pipéridino-6 oxyde-3 pyrimidinyl-4) N′,N′-diméthylurée.

A une suspension agitée à la température ambiante, sous atmosphère inerte, de 50 g de diamino-2,4 pipéridino-6 pyrimidine-oxyde-3 dans 200 cm$^3$ de diméthylsulfoxyde anhydre, on ajoute lentement 1,1 équivalent de chlorure de N,N-diméthylcarbamoyle. Un quart d'heure après la fin de l'addition le milieu réactionnel est homogène et, une heure plus tard le produit formé commence à cristalliser. Après avoir vérifié en chromatographie couche mince la transformation totale du produit de départ, le mélange réactionnel est versé dans 400 cm$^3$ d'eau contenant 1,1 équivalent de soude par rapport à la quantité de chlorure utilisé. La suspension obtenue est refroidie au bain de glace puis elle est saturée de chlorure de sodium.

Le précipitée est essoré, lavé à l'eau puis séché sous pression réduite. On obtient 32 g de solide qui est recristallisé dans le diméthylformamide.

Les 22 g de N-(amino-2 pipéridino-6 oxyde-3 pyrimidinyl-4) N′,N′-diméthylurée, ainsi obtenus sont sous forme de cristaux blancs dont le point de fusion est de 200 ° C.

| Analyse élémentaire : $C_{12}H_{20}N_6O_2$ | | | | |
|---|---|---|---|---|
| | C | H | N | O |
| Calculé | 51,41 | 7,19 | 29,98 | 11,41 |
| Trouvé | 51,29 | 7,20 | 29,78 | 11,70 |

EXEMPLE XIV

Préparation de la N-(amino-2 pipéridino-6 oxyde-3 pyrimidinyl-4) N′,N′-diisopropylurée et de l'amino-7 pipéridino-5-2H-oxadiazolo-1,2,4 [2,3a] pyrimidinone-2

A une suspension agitée à la température ambiante, sous atmosphère inerte, de 102,2 g de diamino-2,4 pipéridino-6 pyrimidine-oxyde-3 dans 500 cm$^3$ de DMSO anhydre, on ajoute goutte à goutte un équivalent de chlorure de N,N-diisopropylcarbamoyle. La réaction est exothermique et la température s'élève de 30 ° C. Le mélange devenu homogène est alors maintenu pendant une heure trente à 50 ° C après la fin de l'addition de chlorure puis abandonné à température ambiante pendant la nuit. Une analyse HPLC de la

solution montre qu'elle ne contient que le chlorure de diamino-2,4 (N,N-diisopropylcarbamoyloxy)-3 pipéridino-6 pyrimidinium.

Le lendemain, le mélange réactionnel de couleur jaune est versé sous agitation dans deux litres d'eau glacée contenant 1 équivalent de soude par rapport au chlorure de carbamoyle utilisé. On obtient un précipité qui par addition de 500 $cm^3$ d'acétate d'éthyle dans la suspension se divise bien. Ce précipité est essoré et séché sous pression réduite à 75 ° C. On obtient à ce stade 95 g d'un mélange dont le produit principal est la N-(amino-2 pipéridino-6 oxyde-3 pyrimidinyl-4) N′,N′-diisopropylurée, le produit secondaire étant l'amino-7 pipéridino-5-2H-oxadiazolo-1,2,4 [2,3a] pyrimidinone-2, formé à partir de la N-(amino-4 pipéridino-6 oxyde-3 pyrimidinyl-2) N′,N′-diisopropylurée instable dans ces conditions de traitement.

Le filtrat est extrait à l'acétate d'éthyle et au butanol.

Ces différentes phases organique sont rassemblées, séchées sur sulfate de sodium. Le solvant est éliminé et on isole ainsi 15 g de mélange dont les proportions sont peu différentes du précipité isolé ci-dessus.

Les 110 g de mélange ainsi obtenu sont extraits sous agitation par 500 $cm^3$ d'éthanol bouillant. L'urée attendue se solubilise, alors que l'amino-7 pipéridino-5-2H-oxadiazolo-1,2,4 [2,3a] pyrimidinone-2 est insoluble. Elle est essorée, puis reprise à nouveau par 200 $cm^3$ d'éthanol bouillant et la suspension est filtrée. On obtient ainsi 30 g d'amino-7 pipéridino-5-2H-oxadiazolo-1,2,4 [2,3a] pyrimidinone-2 sous forme de cristaux blancs dont le point de fusion est de 260 ° C.

| Analyse élémentaire : $C_{10}H_{13}N_5O$ | | | | |
|---|---|---|---|---|
| | C | H | N | O |
| Calculé<br>Trouvé | 51,05<br>51,00 | 5,57<br>5,65 | 29,77<br>29,70 | 13,60<br>13,85 |

Les filtrats éthanoliques précédents sont concentrés, puis glacés. L'urée attendue cristalise; elle est essorée, séchée puis recristallisée une seconde fois dans 100 $cm^3$ d'éthanol. On obtient 35 g de N-(amino-2 pipéridino-6 oxyde-3 pyrimidinyl-4), N′,N-diisopropylurée sous forme d'aiguilles blances dont le point de fusion est de 240 ° C.

| Analyse élémentaire : $C_{16}H_{28}N_6O_2$ | | | | |
|---|---|---|---|---|
| | C | H | N | O |
| Calculé<br>Trouvé | 57,11<br>56,98 | 8,39<br>8,18 | 24,98<br>24,68 | 9,51<br>10,11 |

## EXEMPLE XV

### Préparation de l'amino-7 pipéridino-5-2H-oxadiazolo-1,2,4 [2,3-c] pyrimidinone-2.

Une suspension agitée de 10 g de N-(amino-2 pipéridino-6 oxyde-3 pyrimidinyl-4) N′,N′-diisopropylurée dans 100 $cm^3$ de toluène anhydre est portée sous atmosphère inerte à la température d'ébullition du toluène pendant trois heures. Le milieu réactionnel devient progressivement homogène.

La solution à la fin de la réaction est refroidie dans un bain de glace. Les cristaux sont essorés, lavés à l'acétone froide et séchés. On obtient 6,5 g d'amino-7 pipéridino-5-2H-oxadiazolo-1,2,4 [2,3-c] pyrimidinone-2 sous forme de cristaux blancs de point de fusion de 230 ° C.

| Analyse élémentaire : $C_{10}H_{13}N_5O_2$ | | | | |
|---|---|---|---|---|
| | C | H | N | O |
| Calculé<br>Trouvé | 51,05<br>51,09 | 5,57<br>5,51 | 29,77<br>29,66 | 13,60<br>13,57 |

Ce produit est également préparé à partir de la N-(amino-2 pipéridino-6 oxyde-3 pyrimidinyl-4) N′,N′-

diméthylurée. Une suspension de 0,5 g de cette urée dans 10 $cm^3$ de toluène anhydre est portée à la température d'ébullition du solvant pendant 10 heures, temps au bout duquel toute l'urée de départ est transformée. A aucun moment le milieu est homogène. A température ambiante le solide est essoré, lavé à l'acétone puis séché. On obtient 0,34 g d'amino-7 pipéridino-5-2H-oxadiazolo-1,2,4[2,3-c]pyridinone-2.

EXEMPLE XVI

Préparation de la [(N-n-butylcarbamoyl) amino]-7 pipéridino-5-2H-oxadiazolo-1,2,4[2,3-c]pyrimidinone-2.

A une suspension, agitée sous atmosphère inerte, de 1 g d'amino-7 pipéridino-5-2H-oxadiazolo-1,2,4 [2,3-c] pyrimidinone-2 (préparée suivant l'exemple XV) dans 20 $cm^3$ de diméthylsulfoxyde, on ajoute à température ambiante 1 $cm^3$ de n-butylisocyanate. Puis le mélange est porté sous agitation pendant douze heures à 80 ° C.

Ensuite, il est versé à la température ambiante dans 100 $cm^3$ d'une solution aqueuse de chlorure de sodium. Le précipité formé est essoré, lavé à l'eau et séché sous pression réduite. On obtient 1,3 g de [(N-n-butylcarbamoyl) amino]-7 pipéridino-5-2H-oxadiozolo-1,2,4 [2,3-c] pyrimidinone-2, sous forme de poudre blanche dont le point de fusion est de 252 ° C.

| Analyse élémentaire : $C_{15}H_{22}N_6O_3$ | | | | |
|---|---|---|---|---|
| | C | H | N | O |
| Calculé<br>Trouvé | 53,88<br>53,35 | 6,63<br>6,49 | 25,14<br>24,66 | 14,35<br>14,19 |

EXEMPLE XVII

Préparation de la [(N-n butylcarbamoyl) amino]-7 pipéridino-5-2H-oxadiazolo-1,2,4 [2,3a] pyrimidinone-2

a) A partir de la N-(amino-2 pipéridino-6 oxyde-3 pyrimidinyl-4) N′,N′-diméthylurée.

Un mélange agité sous amosphère inerte de 9,5 g de N-(amino-2 pipéridino-6 oxyde-3 pyrimidinyl-4) N′,N′-diméthylurée et de 4 $cm^3$ de n-butylisocyanate dans 50 $cm^3$ de diméthylsulfoxyde anhydre est porté à une température de 50 ° C pendant dix heures. Le produit de départ étant totalement transformé, le mélange est versé dans l'eau saturée de chlorure de sodium. Le produit précipité est essoré puis séché.

Après recristallisation dans un mélange éthanol-acétonitrile, on obtient 6 g de [(N-n butyl carbamoyl) amino]-7 pipéridino-5-2H-oxadiazolo-1,2,4 [2,3a] pyrimidinone-2 sous forme de cristaux blanc nacré dont le point de fusion est de 221 ° C.

| Analyse élémentaire : $C_{15}H_{22}N_6O_3$ | | | | |
|---|---|---|---|---|
| | C | H | N | O |
| Calculé<br>Trouvé | 53,88<br>53,83 | 6,63<br>6,72 | 25,14<br>25,11 | 14,35<br>14,30 |

b) A partir de l'amino-7 pipéridino-5-2H-oxadiazolo-1,2,4 [2,3a] pyrimidinone-2.

Ce produit est également préparé par addition du n-butylisocyanate sur l'amino-7 pipéridino-5-2H-oxadiazolo-1,2,4 [2,3a] pyrimidinone-2.

A une suspension de 3 g de ce produit dans 100 $cm^3$ de DMSO anhydre on ajoute 3 $cm^3$ de n-butylisocyanate. Le mélange est agité sous atmosphère inerte pendant huit heures à une température comprise entre 80° et 90 ° C. Puis à température ambiante, il est versé dans 400 $cm^3$ d'eau. Le produit précipité est essoré, lavé à l'eau puis à l'acétone. Les cristaux sont séchés et agités dans 150 $cm^3$ de toluène à 50 ° C. Le produit de départ étant instable dans ces conditions, est filtré. Le filtrat est concentré sous pression réduite, puis glacé. La [(N-n-butylcarbamoyl) amino-7] pipéridino-5-2H-oxadiazolo-1,2,4 [2,3a]

pyrimidinone-2 cristallise. On obtient 1,3 g de cristaux beiges dont le point de fusion est de 221 ° C. Le specter $^1$H RMN est conforme à la structure et identique à celui obtenu à partir du produit préparé par la première méthode.

c) A partir de la N-(amino-2 pipéridino-6 oxyde-3 pyrimidinyl-4), N-n-butylurée.

1°/ Synthèse de la N-(pipéridino-6 amino-2 oxyde-3 pyrimidinyl-4),N′-n-butyl urée

A une suspension de 10 g de pipéridino-6 diamino-2,4 pyrimidine oxyde-3 dans 75 $cm^3$ de DMSO portée à 50-60 ° C, on ajoute 6,5 $cm^3$ de n-butylisocyante. L'addition est effectuée par fractions de 1 $cm^3$ pendant 3-4 heures.

Après la dernière addition, le milieu réactionnel devient homogène. On maintient le chauffage pendant environ 2 heures, puis la solution est versée sur environ 1 litre d'eau glacée additionnée d'un peu d'acide acétique. Le produit attendu cristallise. Après neutralisation de la phase aqueuse, le brut réactionnel est filtré et séché sous vide.

Le brut réactionnel est composé du produit attendu ainsi que du produit de dicondensation.

Par purification chromatographique sur gel de silice (éluant : $CH_2Cl_2/MeOH/NH_4OH$), on isole les deux produits.

On obtient 8,5 g de la N-(pipéridino-6 amino-2 oxyde-3 pyrimidinyl-4), N′-n-butyl urée sous forme de cristaux blancs fondant à 202-204 ° C.

| Analyse élémentaire : $C_{14}H_{24}N_6O_2$ | | | | |
|---|---|---|---|---|
| | C% | H% | N% | O% |
| Calculé | 54,50 | 7,80 | 27,27 | 10,38 |
| Trouvé | 54,36 | 7,80 | 27,20 | 10,51 |

2°/ Synthèse de la [(N-n-butylcarbamoyl)amino]-7 pipéridino-5-2H-oxadiazolo-1,2,4[2,3a]pyrimidinone-2

A une suspension agitée sous atmosphère inerte de 1,3 g de cette urée dans 20 $cm^3$ de dioxanne anhydre, on ajoute à température ordinaire 1,1 équivalent de chlorure N,N-diméthylcarbamoyle. Après deux heures d'agitation à température ambiante, le mélange est porté à 80 ° C pendant trois heures. Puis à température ordinaire, le précipité est essoré, lavé à l'eau et séché.

On obtient 0,7 g de N-(N,N-diméthylcarbamoyl amino-2 pipéridino-6 oxyde-3 pyrimidinyl-4) N′-n-butylurée dont le spectre de $^1$H RMN correspond à la structure attendue. Une suspension de ce produit dans 50 $cm^3$ de toluène anhydre est portée au reflux du solvant pendant cinq heures. Par refroidissement, le produit attendu cristallise; il est essoré, lavé au pentane et séché. On obtient 0,400 g de [(N-n-butylcarbamoyl) amino]-7 pipéridino-5-2H-oxadiazolo-1,2,4 [2,3a] pyrimidinone-2.

EXEMPLE XVIII

Préparation de l'amino-2 [bis (N,N-diméthylcarbamoyl) amino]-4 pipéridino-6 pyrimidine-oxyde 3

A une suspension agitée à une température d'environ 50 ° C sous atmosphère inerte de 5 g de N-(amino-2 pipéridino-6 oxyde-3 pyrimidinyl-4) N',N'-diméthylurée dans 50 $cm^3$ de dioxanne anhydre, on ajoute goutte à goutte 1,8 $cm^3$ de chlorure de N,N-diméthylcarbamoyle. Un précipité se forme progressivement. Le mélange est encore agité deux heures après la fin de l'introduction du chlorure, puis glacé. Le solide est essoré puis lavé au dioxanne et séché. Ce produit est versé dans 50 $cm^3$ d'eau.

On obtient une solution homogène à laquelle on ajoute goutte à goutte un équivalent de soude par rapport au chlorure utilisé. Le produit attendu précipite il est essoré et séché. On obtient 5 g d'amino-2 [bis (N,N-diméthylcarbamoyl) amino]-4 pipéridino-6 oxyde-3 sous forme d'une poudre blanche dont le point de fusion est de 175 ° C.

| Analyse élémentaire : $C_{15}H_{25}N_7O_3$ | | | | |
|---|---|---|---|---|
| | C | H | N | O |
| Calculé<br>Trouvé | 51,26<br>51,21 | 7,17<br>6,87 | 27,90<br>28,03 | 13,66<br>13,68 |

EXEMPLES DE FORMULATION

1) On prépare une lotion destinée au traitement de la chute des cheveux de la composition suivante :

| | |
|---|---|
| - N-(amino-2 pyrrolidino-6 oxyde-3 pyrimidinyl-4)N′,N′-diméthylurée (exemple II) | 3,8 g |
| - Propylèneglycol | 20,0 g |
| - Ethanol | 50,0 g |
| - Eau | qsp 100,0 g |

2) On prépare une lotion de la composition suivante :

| | |
|---|---|
| - N-(amino-2 pipéridino-6 oxyde-3 pyrimidinyl-4)N′,N′-diméthylurée (exemple XIII) | 3,8 g |
| - Propylèneglycol | 20,0 g |
| - Ethanol | 50,0 g |
| - Eau | qsp 100,0 g |

3) On prépare une lotion de la composition suivante :

| | |
|---|---|
| - N-(amino-2 pipéridino-6 oxyde-3 pyrimidinyl-4)N′,N′-diisopropylurée (exemple XIV) | 5,2 g |
| - Propylèneglycol | 20,0 g |
| - Ethanol | 50,0 g |
| - Eau | qsp 100,0 g |

4) On prépare une lotion destinée à stimuler la repousse des cheveux de la composition suivante :

| | |
|---|---|
| - Amino-7 pipéridino-5-2H-oxadiazolo-1,2,4[2,3-c]pyrimidinone-2 (exemple XV) | 0,5 g |
| - Propylèneglycol | 6,45 g |
| - Eau | qsp 100,0 g |

5) On prépare une lotion de la composition suivante :

| | |
|---|---|
| - N-(amino-2 pipéridino-6 oxyde-3 pyrimidinyl-4)N′,N′-diméthylurée (exemple XIII) | 3,1 g |
| - Propylèneglycol | 6,45 g |
| - Eau | qsp 100,0 g |

6) On prépare une lotion de la composition suivante :

| | |
|---|---|
| - N-(amino-2 pipéridino-6 oxyde-3 pyrimidinyl-4)N′,N′-diisopropylurée (exemple XIV) | 5,5 g |
| - Propylèneglycol | 6,45 g |
| - Eau | qsp 100,0 g |

1 à 2 ml de ces lotions sont appliqués sur les zones alopéciques du cuir chevelu; ces applications, éventuellement accompagnées par un massage pour favoriser la pénétration, étant effectuées une ou deux fois par jour.

7) On prépare un gel destiné au traitement de la chute des cheveux de la composition suivante :

| | |
|---|---|
| - Amino-7 pipéridino-5-2H-oxadiazolo-1,2,4[2,3-c]pyrimidinone-2 sous forme micronisée (exemple XV) | 5,0 g |
| - Acide acrylique réticulé, PM = 3 millions, vendu par la Société GOODRICH, sous la dénomination commerciale "CARBOPOL 934" | 0,5 g |
| - Propylèneglycol | 4,5 g |
| - Conservateur | 0,15 g |
| - Amino-2 méthyl-2 propanol-1 | qsp pH = 7 |
| - Eau | qsp 100,0 g |

8) On prépare un gel de la composition suivante :

| | |
|---|---|
| - Amino-7 pipéridino-5-2H-oxadiazolo-1,2,4[2,3-a]pyrimidinone-2 sous forme micronisée (exemple XIV) | 5,00 g |
| - Acide acrylique réticulé, PM = 3 millions, vendu par la Société GOODRICH, sous la dénomination commerciale "CARBOPOL 934" | 0,5 g |
| - Propylèneglycol | 4,5 g |
| - Conservateur | 0,15 g |
| - Amino-2 méthyl-2 propanol-1 | qsp pH = 7 |
| - Eau | qsp 100,0 g |

9) On prépare un gel de la composition suivante :

| | |
|---|---|
| - [(N-n-butylcarbamoyl)amino]-7 pipéridino-5-2H-oxadiazolo-1,2,4 [2,3-a]pyrimidinone-2, sous forme micronisée (exemple XVII) | 5,00 g |
| - Acide polyacrylique réticulé, PM = 3 millions, vendu par la Société GOODRICH, sous la dénomination commerciale "CARBOPOL 934" | 0,5 g |
| - Propylèneglycol | 4,5 g |
| - Conservateur | 0,15 g |
| - Amino-2 méthyl-2 propanol-1 | qsp pH = 7 |
| - Eau | qsp 100,0 g |

**Revendications**

**Revendications pour les Etats contractants suivants : AT, BE, CH, DE, FR, GB, IT, LI, NL, SE**

**1.** Composé caractérisé par le fait qu'il répond à la formule générale (I) ou sa forme isomère de formule (II) :

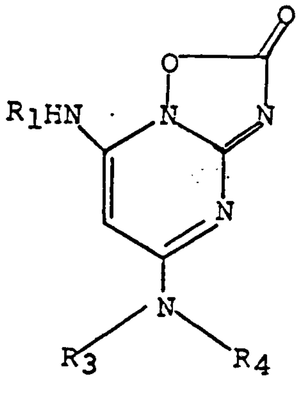
(I)

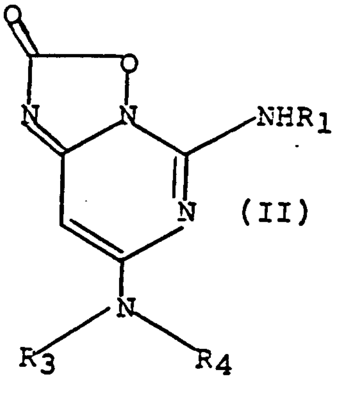
(II)

dans laquelle :

$R_3$ et $R_4$, identiques ou différents, désignent un atome d'hydrogène, un radical alkyle linéaire ou ramifié en $C_1$-$C_{18}$, alcényle ayant 2 à 18 atomes de carbone, cycloalkyle de 5 à 8 atomes de carbone, éventuellement substitués par un ou plusieurs groupements alkyle en $C_1$-$C_6$, les groupements alkyle, alcényle ou cycloalkyle pouvant eux-mêmes être substitués par un ou plusieurs groupement(s) hydroxyle;

$R_3$ ou $R_4$ désignent également un groupement aryle ou aralkyle répondant à la formule (III) :

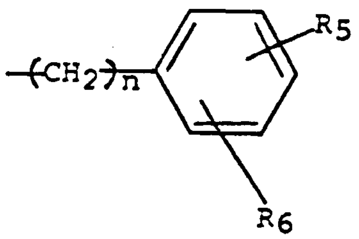

(III)

dans laquelle :

n peut prendre les valeurs de 0 à 4 où $R_5$ et $R_6$, indépendamment l'un de l'autre, désignent un hydrogène, un groupement alkyle en $C_1$-$C_6$, un groupement nitro, hydroxyle, alcoxy ou un atome d'halogène, ou encore un groupement carboxylique, ainsi que ses formes salifiées, esters et amides;

$R_3$ et $R_4$ pris ensemble avec l'atome d'azote auquel ils sont reliés, peuvent former un hétérocycle choisi parmi les groupements morpholino, pipéridino, pyrrolidino, pipérazino, N-alkyl-4' pipérazino, dans lequel le groupement alkyle en position 4' contient de préférence 1 à 6 atomes de carbone dont l'un est éventuellement substitué par un groupement hydroxyle;

$R_1$ désigne un atome d'hydrogène ou un groupement carbamoyle de formule :

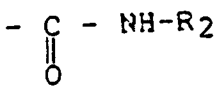

dans laquelle $R_2$ désigne un atome d'hydrogène, un radical alkyle linéaire ou ramifié en $C_1$-$C_{18}$, un groupement alcényle en $C_2$-$C_{18}$, un groupement cycloalkyle en $C_5$-$C_8$, un radical aryle ou aralkyle répondant à la formule (III) ci-dessus, sous la condition que lorsque $R_1$ désigne hydrogène, $R_3$ et $R_4$ pris ensemble avec l'azote auquel ils sont reliés, ne désignent pas [3,6-dihydro-1(2H)-pyridyl]

**2.** Composé selon la revendication 1, caractérisé par le fait que dans la formule (I) ou la formule (II), le radical alkyle en $C_1$-$C_{18}$ est choisi parmi les radicaux méthyle, éthyle, propyle, éthyl-2 hexyle, octyle, dodécyle, hexadécyle et octadécyle, que le radical alkyle inférieur est choisi parmi les groupements méthyle, éthyle, isopropyle, butyle et tertiobutyle, que les groupements alcényle en $C_2$-$C_{18}$ sont choisis parmi les groupements allyle, butényle, hexényle, dodécényle, hexadécényle et octadécényle, que les groupements aryle ou aralkyle sont choisis parmi les radicaux phényle, tolyl-4, nitro-2 phényle, nitro-4phényle, fluoro-4 phényle, chloro-4 phényle, carboxy-2 phényle, carboxy-4 phényle, hydroxy-4 phényle, benzyle, phénéthyle.

**3.** Composé selon la revendication 1 ou 2, caractérisé par le fait qu'il répond à la formule (Ia) ou sa forme isomère de formule (IIa) :

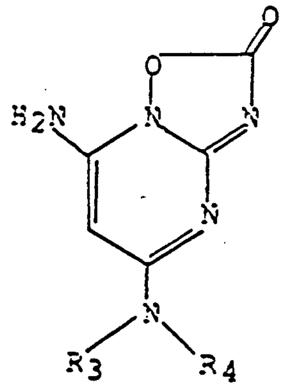

(Ia)

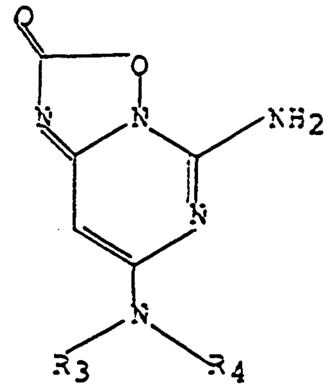

(IIa)

dans laquelle $R_3$ et $R_4$ ont la signification indiquée dans la revendication 1.

**4.** Composé selon la revendication 1 ou 2, caractérisé par le fait que le composé répond à la formule (Ib) ou sa forme isomère (IIb) :

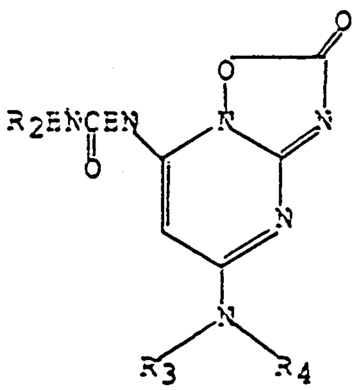

(Ib)

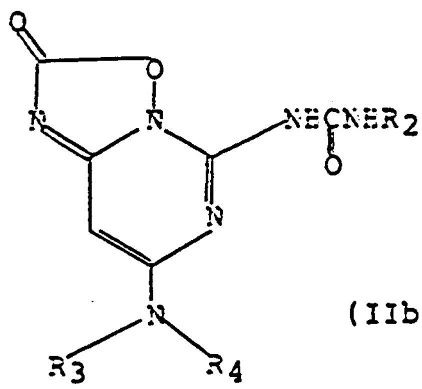

(IIb)

dans lesquelles $R_2$, $R_3$ et $R_4$ ont la signification indiquée dans la revendication 1.

**5.** Composé selon la revendication 4, caractérisé par le fait qu'il répond à la formule (Ic) ou sa forme isomère (IIc) :

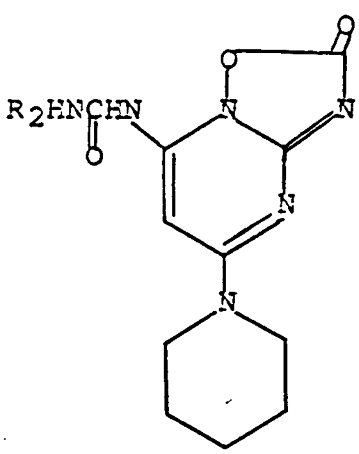

(Ic)

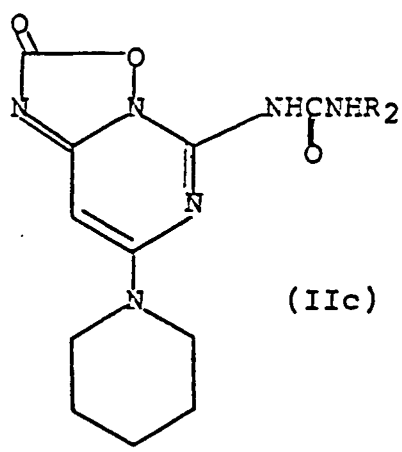

(IIc)

dans laquelle $R_2$ a la signification indiquée dans la revendication 1.

**6.** Composé caractérisé par le fait qu'il répond à la formule (VIIa) ou à sa forme isomère (VIIb) :

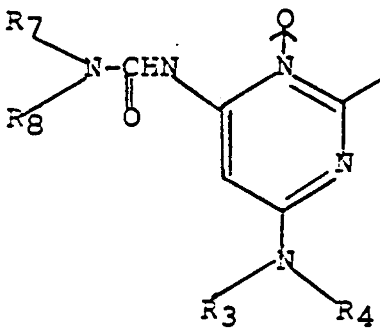

(VIIa)

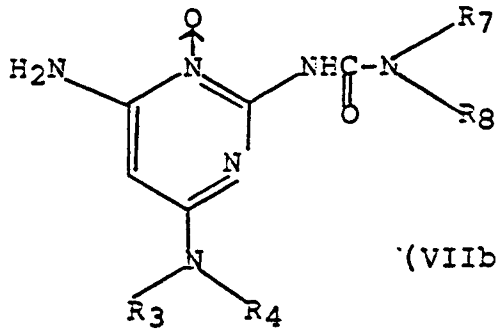

(VIIb)

dans laquelle $R_3$ et $R_4$ ont la même signification que celle indiquée dans la revendication 1, et $R_7$ et $R_8$, indépendamment l'un de l'autre, désignent un radical alkyle linéaire ou ramifié en $C_1$-$C_{18}$, un groupement alcényle en $C_2$-$C_{18}$, un groupement cycloalkyle en $C_5$-$C_8$, un groupement aryle ou aralkyle répondant à la formule (III) définie dans la revendication 1.

**7.** Procédé de préparation du composé selon la revendication 1 ou 2, en particulier les composés répondant à la formule (Ia) ou (IIa) :

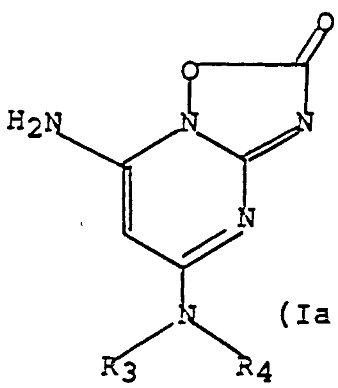

(Ia)

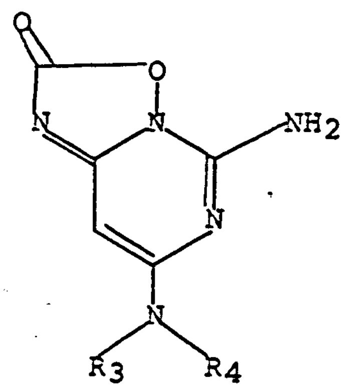

(IIa)

caractérisé par le fait que l'on traite la monourée de la triamino-2,4,6 pyrimidine oxyde-3 de formule (VIIa) ou sa forme isomère de formule (VIIb) :

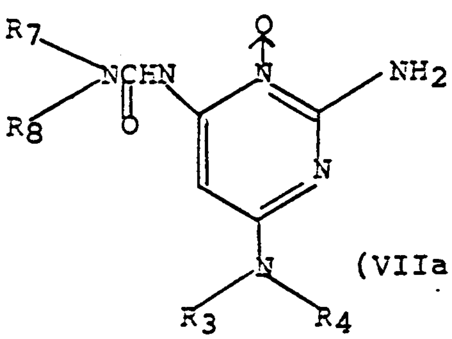

(VIIa)

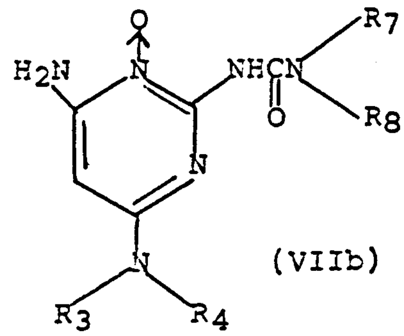

(VIIb)

dans laquelle $R_3$, $R_4$, $R_7$ et $R_8$ ont la signification indiquée dans la revendication 6 par une réaction d'élimination-cyclisation en présence d'un solvant organique, en portant la monourée (VIIb) à une température comprise entre 40° et 100°C pour obtenir le composé de formule (Ia) ou la monourée (VIIa) à une température supérieure à 100°C pendant quelques heures pour obtenir le composé de formule (IIa).

**8.** Composé caractérisé par le fait qu'il répond à la formule (VI) :

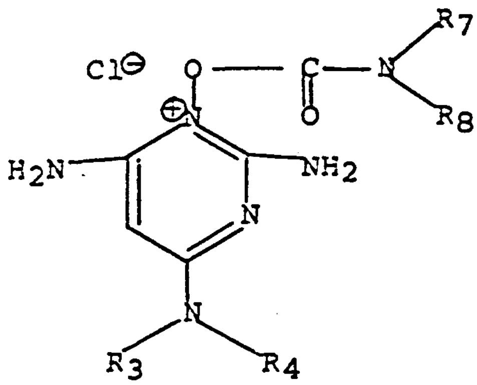

(VI)

dans laquelle $R_3$, $R_4$, $R_7$ et $R_8$ ont la signification indiquée dans la revendication 6.

**9.** Procédé de préparation selon la revendication 7, caractérisé par le fait que les composés de formule (VIIa) et leurs isomères de formule (VIIb) sont préparés en traitant la triamino-2,4,6 pyrimidine oxyde-3 de formule (IV) :

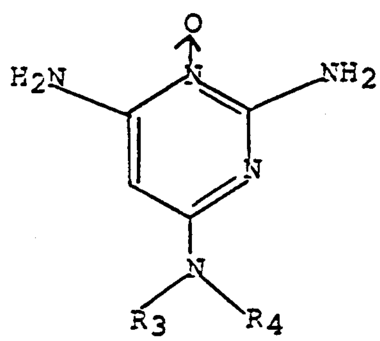

(IV)

dans laquelle $R_3$ et $R_4$ ont la signification indiquée dans la revendication 1, par un chlorure de carbamoyle de formule (V) :

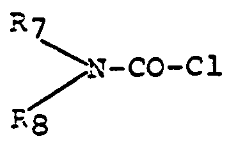

(V)

dans laquelle $R_7$ et $R_8$ ont la signification indiquée dans la revendication 6, par un solvant aprotique polaire pour obtenir le chlorure de formule (VI) suivante :

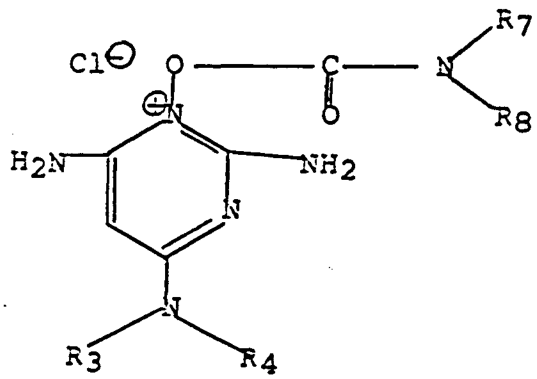

(VI)

que l'on traite ensuite par un équivalent de base forte par rapport au chlorure de carbamoyle utilisé.

**10.** Procédé de préparation des composés selon la revendication 4, répondant à la formule (Ib) ou sa forme isomère (IIb) :

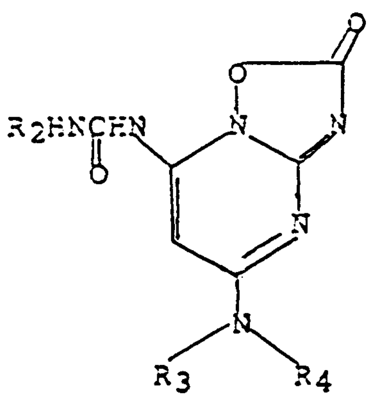

(Ib)

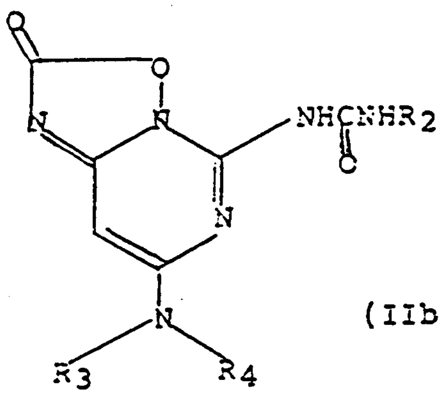

(IIb)

caractérisé par le fait que l'on traite l'urée de formule (VIIa) ou (VIIb) par un isocyanate de formule (IX) :

$R_2\text{-}N=C=O$ (IX)

dans laquelle $R_2$ a la signification indiquée dans la revendication 1, dans le diméthylsulfoxyde (DMSO).

**11.** Procédé de préparation des composés selon la revendication 4, de formule (Ib) ou (IIb), caractérisé par le fait que l'on traite directement l'oxadiazolo pyrimidinone de formule (Ia) ou son isomère de formule (IIa) :

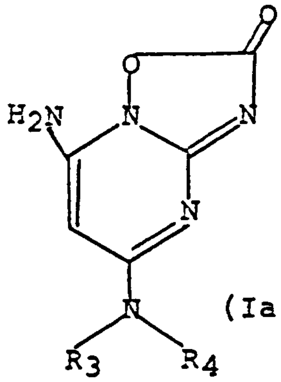

(Ia)

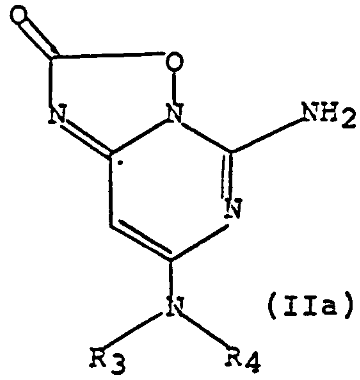

(IIa)

par l'isocyanate de formule (IX), dans un solvant polaire aprotique.

**12.** Procédé de préparation du composé de formule (Ib) selon la revendication 4, caractérisé par le fait que l'on traite une monourée de formule (Xa) :

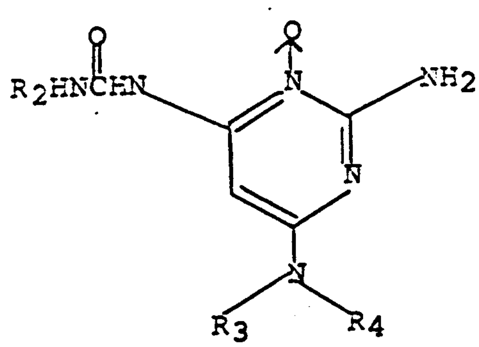

(Xa)

dans laquelle $R_2$, $R_3$ et $R_4$ ont la signification indiquée dans la revendication 1, dans le dioxanne par un chlorure de carbamoyle de formule (V) telle que définie dans la revendication 9 pour obtenir la diurée de formule (XIa) :

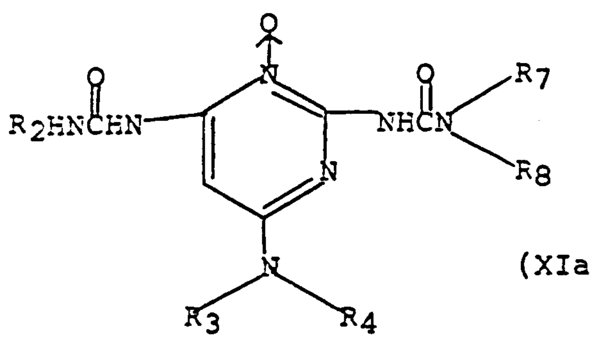

(XIa)

que l'on porte ensuite à une température comprise entre 50 et 120°C pendant quelques heures en présence d'un solvant organique.

**13.** Composé caractérisé par le fait qu'il répond à la formule (VIIIa) :

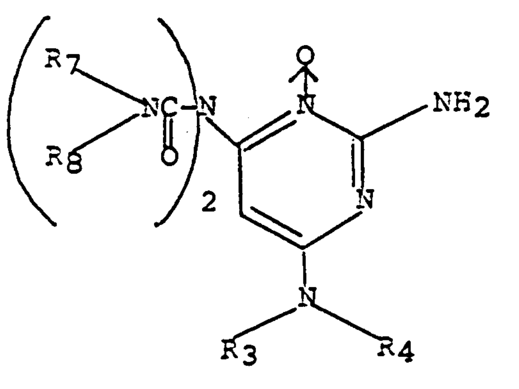

(VIIIa)

dans laquelle $R_3$, $R_4$, $R_7$ et $R_8$ ont les significations indiquées dans la revendication 6.

**14.** Composé caractérisé par le fait qu'il répond à la formule (XIa) :

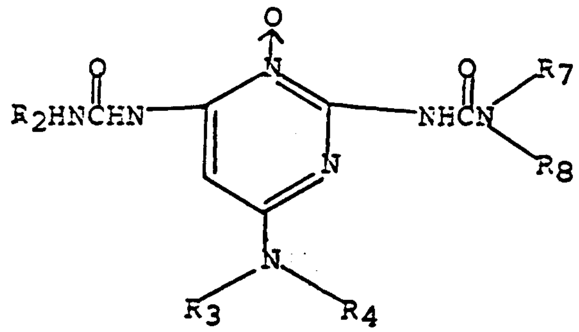

(XIa)

dans laquelle $R_2$, $R_3$, $R_4$, $R_7$, $R_8$ ont les significations définies dans les revendications 1 à 6.

**15.** Composition cosmétique ou pharmaceutique, caractérisée par le fait qu'elle contient, dans un support approprié pour une application topique, au moins un composé répondant à l'une quelconque des revendications 1 à 5.

**16.** Composition selon la revendication 15, caractérisée par le fait qu'elle se présente sous forme d'onguents, de teintures, de crèmes, de pommades, de poudres, de timbres, de tampons imbibés, de solutions, d'émulsions, de lotions, de gels, de sprays ou de suspensions anhydres ou aqueuses.

**17.** Composition pharmaceutique selon la revendication 15, caractérisée par le fait que le composé de formule (I) est présent dans des concentrations comprises entre 0,1 et 10% en poids par rapport au poids total de la composition, en particulier entre 0,2 et 5% en poids.

**18.** Composition cosmétique selon la revendication 15, sous forme de lotions, de gels, de savons, de shampooings, de mousses, caractérisée par le fait qu'elle contient dans un support physiologiquement acceptable, au moins un des composés tels que définis dans l'une quelconque des revendications 1 à 5, à une concentration comprise entre 0,01 et 5% en poids.

**19.** Composition selon la revendication 18, caractérisée par le fait qu'elle contient en plus des agents hydratants, des agents anti-séborréïques et des agents favorisant la repousse des cheveux, des agents anti-inflammatoires stéroïdiens ou non stéroïdiens, des caroténoïdes, les acides eicosatétraynoïque-5,8,11,14 et eicosatriynoïque-5,8,11 et leurs esters et amides.

**20.** Composition selon l'une quelconque des revendications 15 à 19, caractérisée par le fait qu'elle contient également des agents conservateurs, des agents stabilisants et des agents régulateurs de pH, des agents modificateurs de pression osmotique, des agents émulsifiants, des filtres UV-A et UV-B, des agents antioxydants.

**21.** Procédé de traitement cosmétique des cheveux, caractérisé par le fait qu'on applique sur le cuir chevelu au moins une composition telle que définie dans l'une quelconque des revendications 15, 16 et 18 à 20.

**22.** Utilisation des composés selon l'une quelconque des revendications 1 à 5, pour la préparation d'un médicament pour le traitement de la pelade, de la chute des cheveux, de la dermatite desquamante.

**Revendications pour l'Etat contractant suivant : ES**

**1.** Procédé de préparation du composé qui répond à la formule générale (I) ou sa forme isomère de formule (II) :

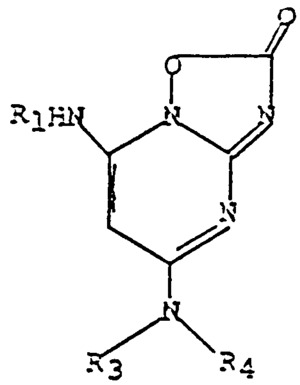

(I)

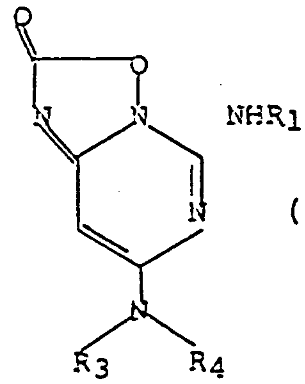

(II)

dans laquelle :

$R_3$ et $R_4$, identiques ou différents, désignent un atome d'hydrogène, un radical alkyle linéaire ou ramifié en $C_1$-$C_{18}$, alcényle ayant 2 à 18 atomes de carbone, cycloalkyle de 5 à 8 atomes de carbone, éventuellement substitués par un ou plusieurs groupements alkyle en $C_1$-$C_6$, les groupements alkyle, alcényle ou cycloalkyle pouvant eux-mêmes être substitués par un ou plusieurs groupement(s) hydroxyle;

$R_3$ ou $R_4$ désignent également un groupement aryle ou aralkyle répondant à la formule (III) :

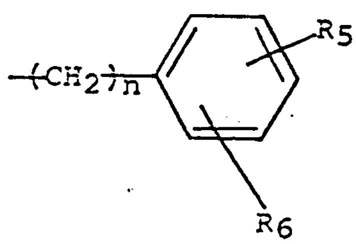

(III)

dans laquelle :

n peut prendre les valeurs de 0 à 4 où $R_5$ et $R_6$, indépendamment l'un de l'autre, désignent un hydrogène, un groupement alkyle en $C_1$-$C_6$, un groupement nitro, hydroxyle, alcoxy ou un atome d'halogène, ou encore un groupement carboxylique, ainsi que ses formes salifiées, esters et amides;

$R_3$ et $R_4$ pris ensemble avec l'atome d'azote auquel ils sont reliés, peuvent former un hétérocycle choisi parmi les groupements morpholino, pipéridino, pyrrolidino, pipérazino, N-alkyl-4' pipérazino, dans lequel le groupement alkyle en position 4' contient de préférence 1 à 6 atomes de carbone dont l'un est éventuellement substitué par un groupement hydroxyle;

$R_1$ désigne un atome d'hydrogène ou un groupement carbamoyle de formule :

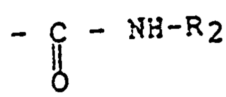

dans laquelle $R_2$ désigne un atome d'hydrogène, un radical alkyle linéaire ou ramifié en $C_1$-$C_{18}$, un groupement alcényle en $C_2$-$C_{18}$, un groupement cycloalkyle en $C_5$-$C_8$, un radical aryle ou aralkyle répondant à la formule (III) ci-dessus, caractérisé par le fait que :

(A) pour préparer les composés répondant à la formule (Ia) ou sa forme isomère de formule (IIa) :

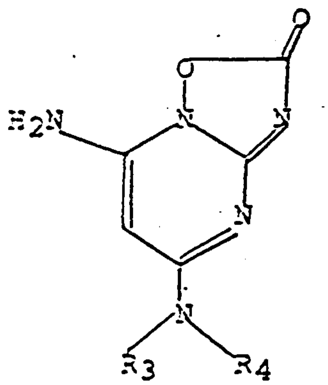

(Ia)

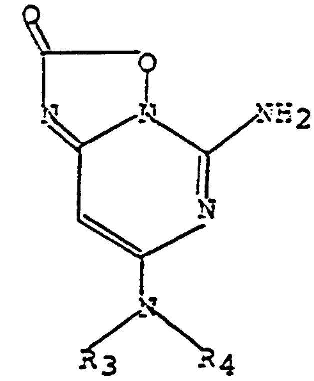

(IIa)

dans laquelle $R_3$ et $R_4$ ont la signification indiquée ci-dessus, l'on traite la monourée de la triamino-2,4,6 pyrimidine oxyde-3 de formule (VIIa) ou sa forme isomère de formule (VIIb) :

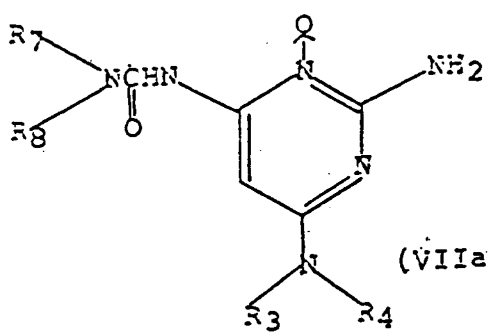

(VIIa)

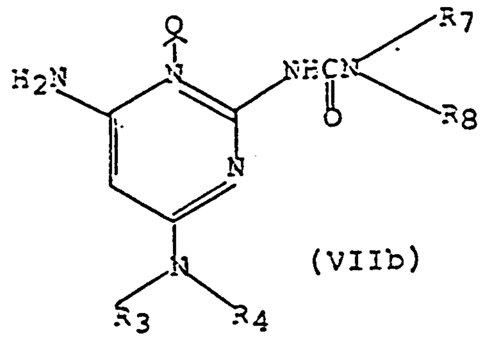

(VIIb)

dans laquelle $R_3$ et $R_4$ ont la même signification que celle indiquée ci-dessus et $R_7$ et $R_8$, indépendamment l'un de l'autre, désignent un radical alkyle linéaire ou ramifié en $C_1$-$C_{18}$, un groupement alcényle en $C_2$-$C_{18}$, un groupement cycloalkyle en $C_5$-$C_8$, un groupement aryle ou aralkyle répondant à la formule (II) définie ci-dessus par une réaction d'élimination-cyclisation en présence d'un solvant organique, en portant à une température comprise entre 40° et 100°C la monourée de formule (VIIb) pour obtenir le composé de formule (Ia) ou la monourée (VIIa) à une température supérieure à 100°C pendant quelques heures pour obtenir le composé de formule (IIa);
(B) pour préparer les composés répondant à la formule (Ib) ou sa forme isomère de formule (IIb) :

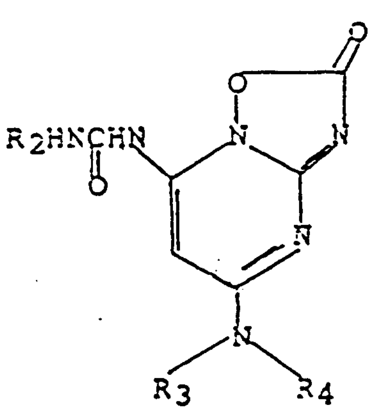

(Ib)

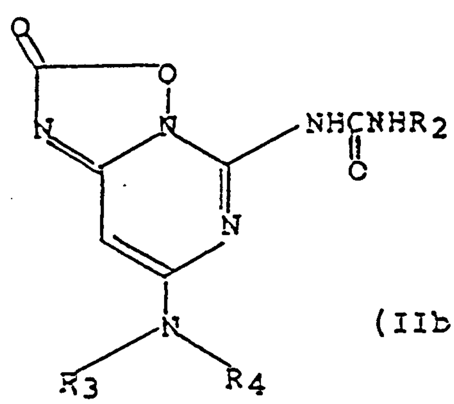

(IIb)

on traite l'urée de formule (VIIa) ou (VIIb) par un isocyanate de formule (IX) :

$R_2$-N=C=O (IX)

dans laquelle $R_2$ a la signification indiquée dans la revendication 1, dans le diméthylsulfoxyde

(DMSO);

(C) pour préparer le composé répondant à la formule (Ib) ou (IIb) telle que définie ci-dessus, l'on traite directement l'oxadiazolo pyrimidinone de formule (Ia) ou son isomère de formule (IIa) :

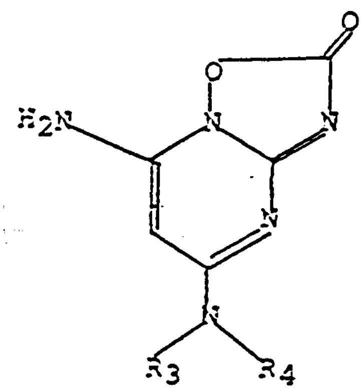

(Ia)

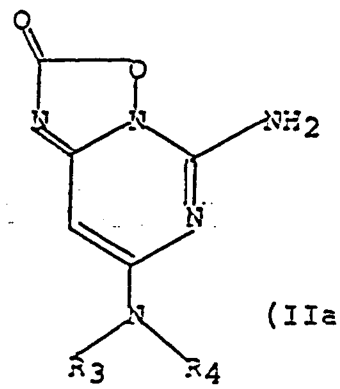

(IIa)

dans laquelle $R_3$ et $R_4$ ont la même signification que celle indiquée ci-dessus, par l'isocyanate de formule (IX) définie ci-dessus, dans un solvant polaire aprotique;

(D) pour préparer les composés répondant à la formule (Ib) définie ci-dessus, l'on traite une monourée de formule (Xa) :

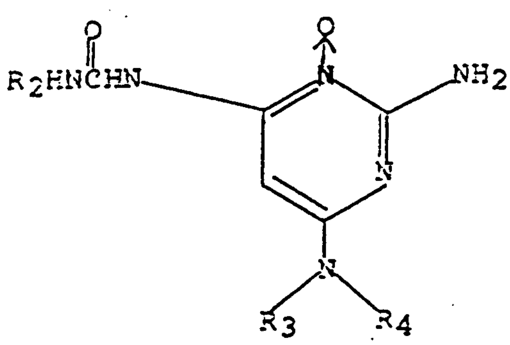

(Xa)

dans laquelle $R_2$ $R_3$ et $R_4$ ont la même signification indiquée ci-dessus dans le dioxanne par un chlorure de carbamoyle de formule (V) :

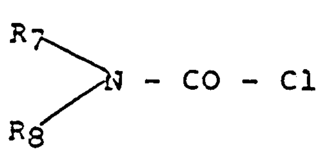

(V)

dans laquelle $R_7$ et $R_8$ ont la signification indiquée ci-dessus, pour obtenir la diurée de formule (XIa) :

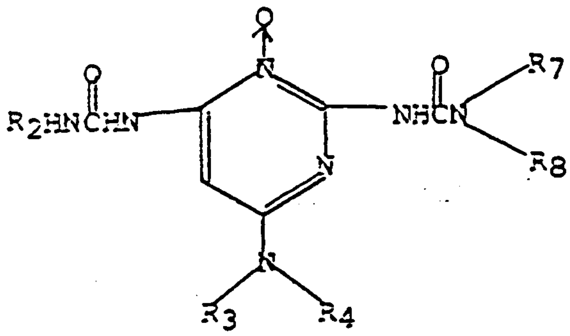

(XIa)

dans laquelle $R_2$, $R_3$, $R_4$, $R_7$ et $R_8$ ont la même signification indiquée ci-dessus, que l'on porte ensuite à une température comprise entre 50 et 120 ° C pendant quelques heures en présence d'un solvant organique.

**2.** Procédé de préparation selon la revendication 1(A), caractérisé par le fait que les composés de formule (VIIa) et leurs isomères de formule (VIIb) sont préparés en traitant la triamino-2,4,6 pyrimidine oxyde-3 de formule (IV) :

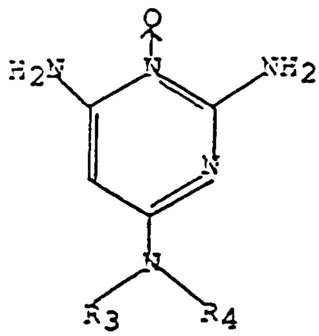

(IV)

dans laquelle $R_3$ et $R_4$ ont la signification indiquée dans la revendication 1, par un chlorure de carbamoyle de formule (V) :

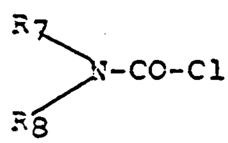

(V)

dans laquelle $R_7$ et $R_8$ ont la signification indiquée dans la revendication 1, par un solvant aprotique polaire pour obtenir le chlorure de formule (VI) suivante :

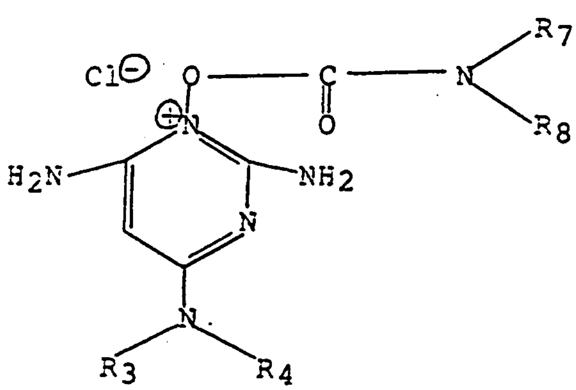

(VI)

que l'on traite ensuite par un équivalent de base forte par rapport au chlorure de carbamoyle utilisé.

3. Procédé selon la revendication 1, caractérisé par le fait que dans la formule (I) ou la formule (II), le radical alkyle en $C_1$-$C_{18}$ est choisi parmi les radicaux méthyle, éthyle, propyle, éthyl-2 hexyle, octyle, dodécyle, hexadécyle et octadécyle, que le radical alkyle inférieur est choisi parmi les groupements méthyle, éthyle, isopropyle, butyle et tertiobutyle, que les groupements alcényle en $C_2$-$C_{18}$ sont choisis parmi les groupements allyle, buténylе, hexényle, dodécényle, hexadécényle et octadécényle, que les groupements aryle ou aralkyle sont choisis parmi les radicaux phényle, tolyl-4, nitro-2 phényle, nitro-4 phényle, fluoro-4 phényle, chloro-4 phényle, carboxy-2 phényle, carboxy-4 phényle, hydroxy-4 phényle, benzyle, phénéthyle.

4. Composition sous forme d'onguents, de teintures, de crèmes, de pommades, de poudres, de timbres, de tampons imbibés, de solutions, d'émulsions, de lotions, de gels, de sprays ou de suspensions anhydres ou aqueuses, caractérisée par le fait qu'elle contient dans un support approprié pour une application topique, au moins un composé obtenu par le procédé d'une quelconque des revendications 1 à 3, à une concentration comprise entre 0,1 et 10% en poids par rapport au poids total de la composition, en particulier entre 0,2 et 5% en poids.

5. Composition cosmétique sous forme de lotions, de gels, de savons, de shampooings, de mousses, caractérisée par le fait qu'elle contient dans un support approprié pour une application topique, au moins un des composés tels que celui obtenu par le procédé d'une quelconque des revendications 1 à 3, à une concentration comprise entre 0,01 et 5% en poids.

6. Composition selon la revendication 5, caractérisée par le fait qu'elle contient en plus des agents hydratants, des agents anti-séborréïques et des agents favorisant la repousse des cheveux, des agents anti-inflammatoires stéroïdiens ou non stéroïdiens, des caroténoïdes, les acides eicosatétraynoïque-5,8,11,14 et eicosatriynoïque-5,8,11 et leurs esters et amides.

7. Composition selon l'une quelconque des revendications 4 à 6, caractérisée par le fait qu'elle contient également des agents conservateurs, des agents stabilisants et des agents régulateurs de pH, des agents modificateurs de pression osmotique, des agents émulsifiants, des filtres UV-A et UV-B, des agents antioxydants.

8. Procédé de traitement cosmétique des cheveux, caractérisé par le fait qu'on applique sur le cuir chevelu au moins une composition telle que définie dans l'une quelconque des revendications 5 à 7.

**Claims**

**Claims for the following Contracting States : AT, BE, CH, DE, FR, GB, IT, LI, NL, SE**

1. Compound, characterised in that it corresponds to the general formula (I) or its isomeric form of formula (II):

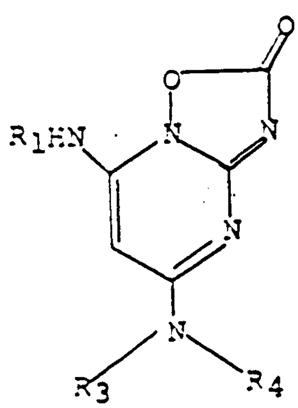

(I)

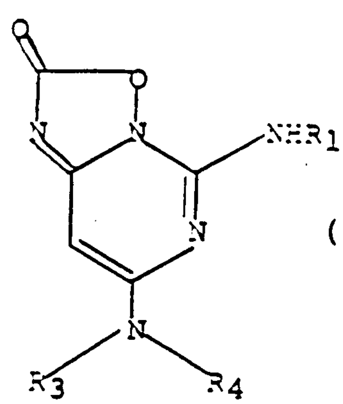

(II)

in which:

$R_3$ and $R_4$, which may be identical or different, denote a hydrogen atom, a linear or branched $C_1$-

$C_{18}$ alkyl radical, an alkenyl radical having 2 to 18 carbon atoms or a cycloalkyl radical having 5 to 8 carbon atoms, these radicals optionally being substituted with one or more $C_1$-$C_6$ alkyl groups, it being possible for the alkyl, alkenyl or cycloalkyl groups themselves to be substituted with one or more hydroxyl group(s);

$R_3$ or $R_4$ also denotes an aryl and aralkyl group corresponding to the formula (III):

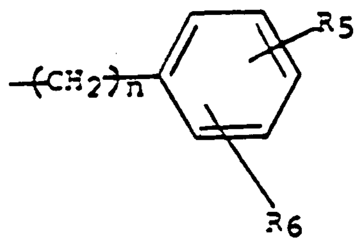

(III)

in which:

n can take values from 0 to 4, where $R_5$ and $R_6$, independently of one another, denote a hydrogen, a $C_1$-$C_6$alkyl group, a nitro, hydroxyl or alkoxy group, or a halogen atom, alternatively a carboxyl group as well as its salified ester and amide forms;

$R_3$ and $R_4$, taken together with the nitrogen atom to which they are attached, can form a heterocycle chosen from morpholino, piperidino, pyrrolidino and piperazino groups and a 4'-(N-alkyl)-piperazino group in which the alkyl group at position 4' preferably contains 1 to 6 carbon atoms, one of which is optionally substituted with a hydroxyl group;

$R_1$ denotes a hydrogen atom or a carbamoyl group of formula:

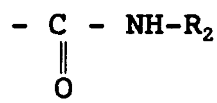

in which $R_2$ denotes a hydrogen atom, a linear or branched $C_1$-$C_{18}$ alkyl radical, a $C_2$-$C_{18}$ alkenyl group, a $C_5$-$C_8$ cycloalkyl group or an aryl or aralkyl radical corresponding to the formula (III) above, with the proviso that, when $R_1$ denotes hydrogen, $R_3$ and $R_4$, taken together with the nitrogen to which they are attached, do not denote [3,6-dihydro-1(2H)-pyridyl].

**2.** Compound according to Claim 1, characterised in that, in the formula (I) or the formula (II), the $C_1$-$C_{18}$ alkyl radical is chosen from methyl, ethyl, propyl, 2-ethylhexyl, octyl, dodecyl, hexadecyl and octadecyl radicals, in that the lower alkyl radical is chosen from methyl, ethyl, isopropyl, butyl and tert-butyl groups, in that the $C_2$-$C_{18}$ alkenyl groups are chosen from allyl, butenyl, hexenyl, dodecenyl, hexadecenyl and octadecenyl groups and in that the aryl or aralkyl groups are chosen from phenyl, 4-tolyl, 2-nitrophenyl, 4-nitrophenyl, 4-fluorophenyl, 4-chlorophenyl, 2-carboxyphenyl, 4-carboxyphenyl, 4-hydroxyphenyl, benzyl and phenethyl radicals.

**3.** Compound according to Claim 1 or 2, characterised in that it corresponds to the formula (Ia) or its isomeric form of formula (IIa):

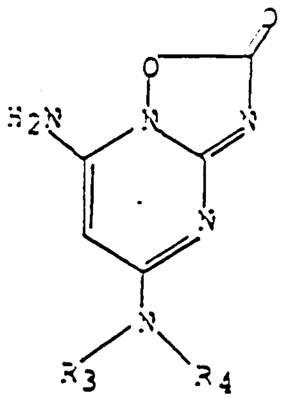

(Ia)

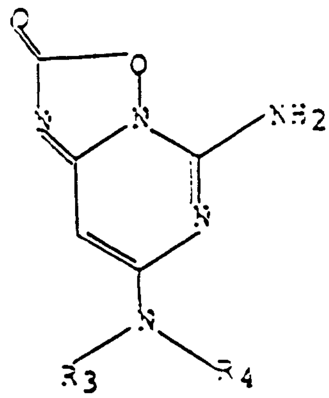

(IIa)

in which $R_3$ and $R_4$ have the meaning stated in Claim 1.

**4.** Compound according to Claim 1 or 2, characterised in that the compound corresponds to the formula (Ib) or its isomeric form (IIb):

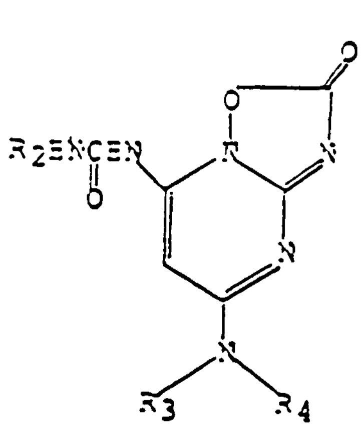

(Ib)

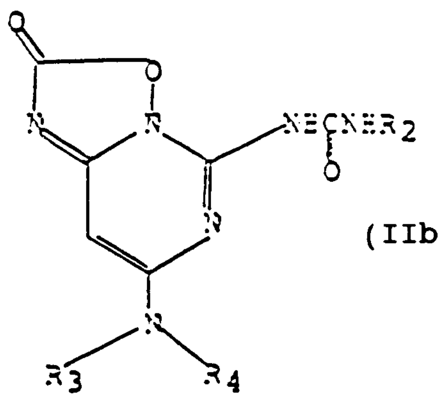

(IIb)

in which $R_2$, $R_3$ and $R_4$ have the meaning stated in Claim 1.

**5.** Compound according to Claim 4, characterised in that it corresponds to the formula (Ic) or its isomeric form (IIc):

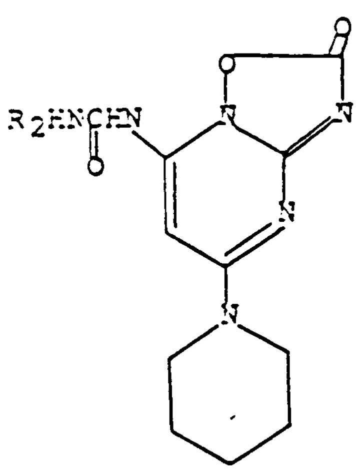

(Ic)

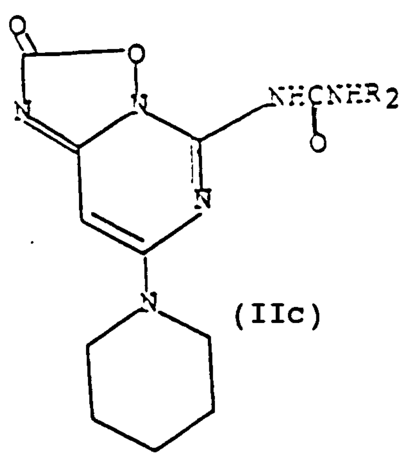

(IIc)

in which $R_2$ has the meaning stated in Claim 1.

**6.** Compound, characterised in that it corresponds to the formula (VIIa) or to its isomeric form (VIIb):

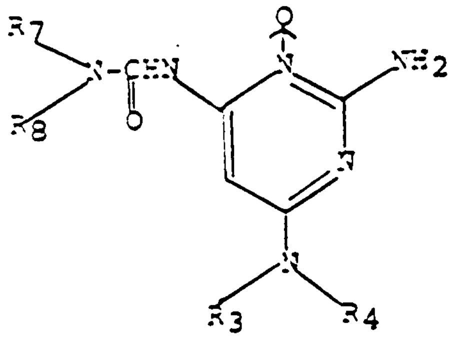

(VIIa)

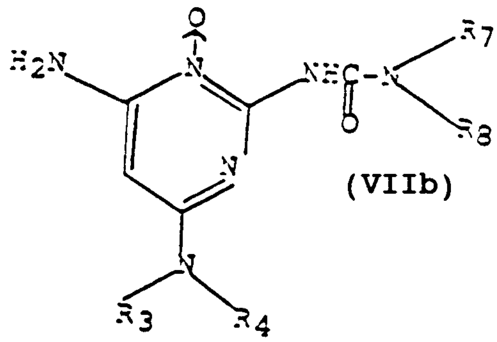

(VIIb)

in which $R_3$ and $R_4$ have the same meaning as stated in Claim 1, and $R_7$ and $R_8$, independently of one another, denote a linear or branched $C_1$-$C_{18}$ alkyl radical, a $C_2$-$C_{18}$ alkenyl group, a $C_5$-$C_8$ cycloalkyl group or an aryl or aralkyl group corresponding to the formula (III) defined in Claim 1.

**7.** Process for preparing the compound according to Claim 1 or 2, especially the compounds corresponding to the formula (Ia) or (IIa):

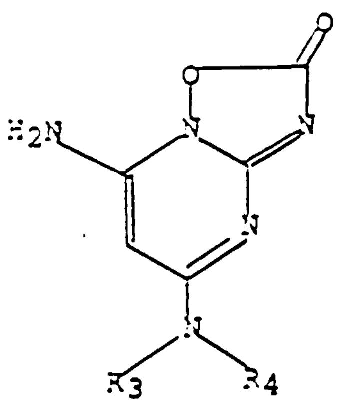

(Ia)

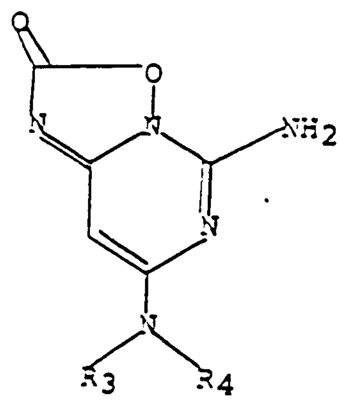

(IIa)

characterised in that the monourea of the 2,4,6-triaminopyrimidine 3-oxide of formula (VIIa) or its isomeric form of formula (VIIb):

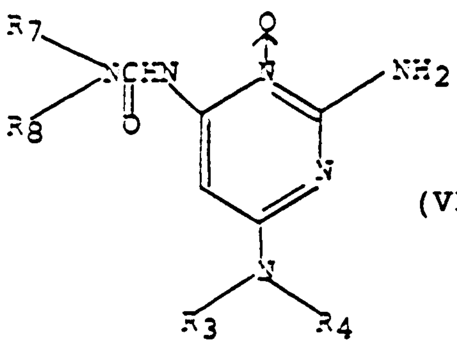

(VIIa)

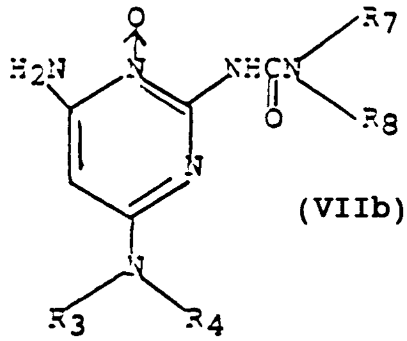

(VIIb)

in which $R_3$, $R_4$, $R_7$ and $R_8$ have the meaning stated in Claim 6, is treated by an elimination/cyclisation reaction in the presence of an organic solvent, bringing the monourea (VIIb) to a temperature of between 40° and 100°C to obtain the compound of formula (Ia), or the monourea (VIIa) to a temperature above 100°C for a few hours to obtain the compound of formula (IIa).

**8.** Compound, characterised in that it corresponds to the formula (VI):

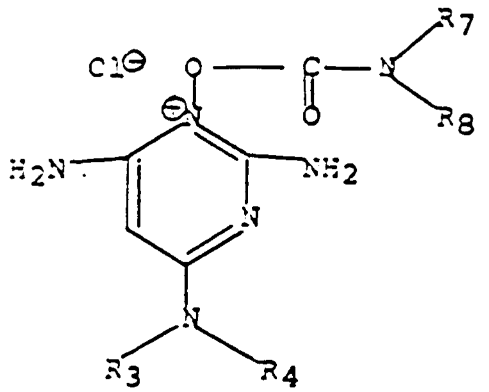

(VI)

in which $R_3$, $R_4$, $R_7$ and $R_8$ have the meaning stated in Claim 6.

**9.** Preparation process according to Claim 7, characterised in that the compounds of formula (VIIa) and their isomers of formula (VIIb) are prepared by treating the 2,4,6-triaminopyrimidine 3-oxide of formula (IV):

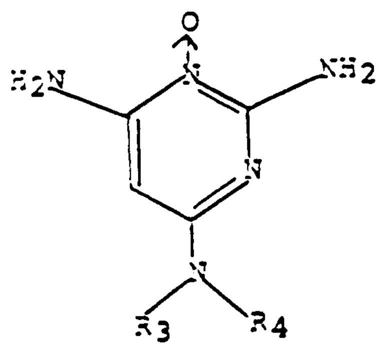

(IV)

in which $R_3$ and $R_4$ have the meaning stated in Claim 1, with a carbamoyl chloride of formula (V):

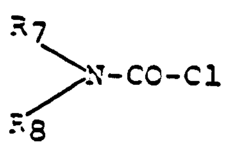

(V)

in which $R_7$ and $R_8$ have the meaning stated in Claim 6, with a polar aprotic solvent, to obtain the chloride of the following formula (VI):

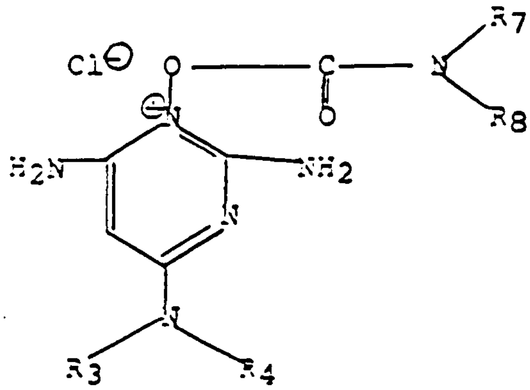

(VI)

which is then treated with one equivalent of strong base with respect to the carbamoyl chloride used.

**10.** Process for preparing the compounds according to Claim 4, corresponding to the formula (Ib) or its isomeric form (IIb):

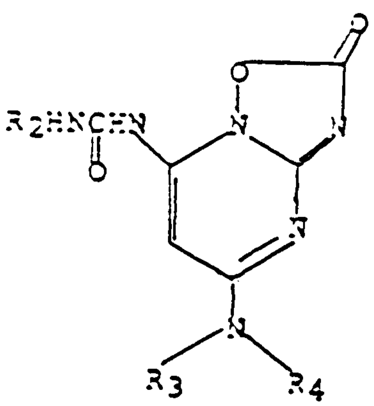

(Ib)

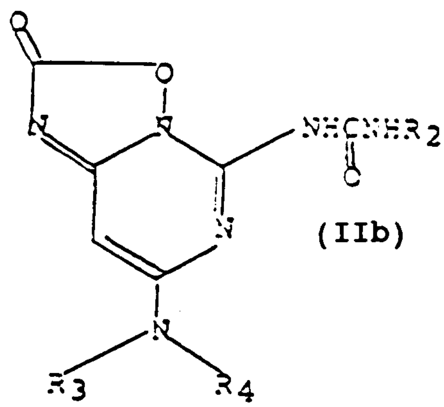

(IIb)

characterised in that the urea of formula (VIIa) or (VIIb) is treated with an isocyanate of formula (IX):

$R_2$-N = C = O (IX)

in which $R_2$ has the meaning stated in Claim 1, in dimethyl sulphoxide (DMSO).

**11.** Process for preparing the compounds according to Claim 4, of formula (Ib) or (IIb), characterised in that the oxadiazolopyrimidinone of formula (Ia) or its isomer of formula (IIa):

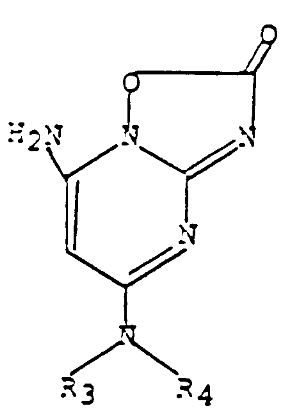

(Ia)

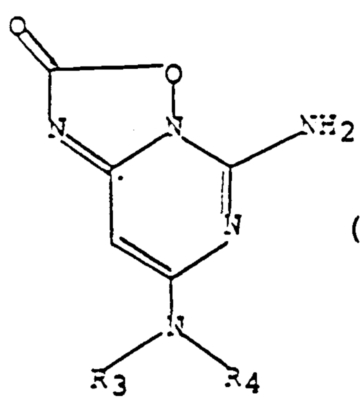

(IIa)

is treated directly with the isocyanate of formula (IX), in an aprotic polar solvent.

**12.** Process for preparing the compound of formula (Ib) according to Claim 4, characterised in that a monourea of formula (Xa):

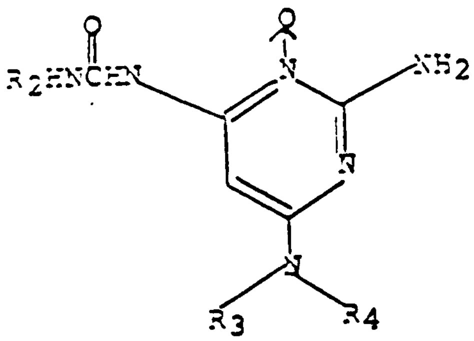

(Xa)

in which $R_2$, $R_3$ and $R_4$ have the meaning stated in Claim 1, is treated in dioxane with a carbamoyl chloride of formula (V) as defined in Claim 9, to obtain the diurea of formula (XIa):

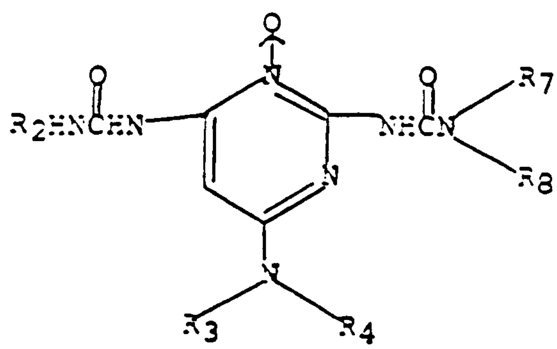

(XIa)

which is then brought to a temperature of between 50 and 120 °C for a few hours in the presence of an organic solvent.

**13.** Compound, characterised in that it corresponds to the formula (VIIIa):

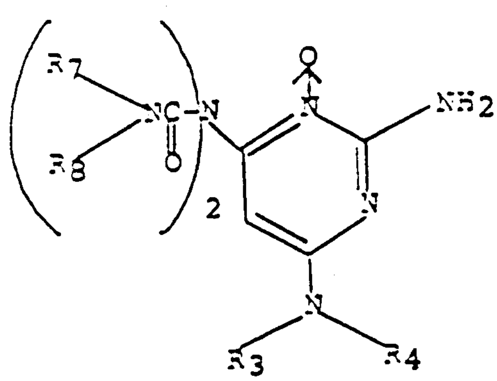

(VIIIa)

in which $R_3$, $R_4$, $R_7$ and $R_8$ have the meanings stated in Claim 6.

**14.** Compound, characterised in that it corresponds to the formula (XIa):

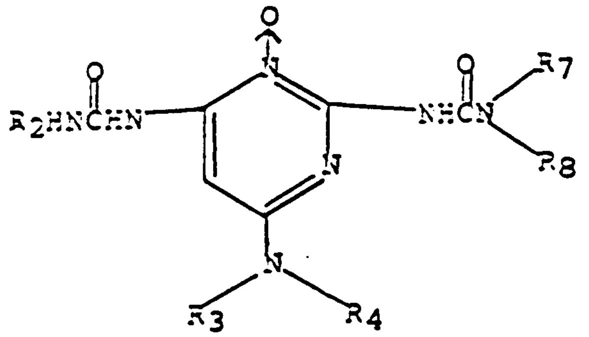

(XIa)

in which $R_2$, $R_3$, $R_4$, $R_7$, and $R_8$ have the meanings defined in Claims 1 to 6.

**15.** Cosmetic or pharmaceutical composition, characterized in that it contains at least one compound corresponding to any one of Claims 1 to 5, in a vehicle suitable for topical application.

**16.** Composition according to Claim 15, characterised in that it is presented in the form of ointments, tinctures, creams, pomades, powders, patches, impregnated pads, solutions, emulsions, lotions, gels, sprays or anhydrous or aqueous suspensions.

**17.** Pharmaceutical composition according to Claim 15, characterised in that the compound of formula (I) is present in concentrations of between 0.1 and 10 % by weight relative to the total weight of the composition, especially between 0.2 and 5 % by weight.

**18.** Cosmetic composition according to Claim 15, in the form of lotions, gels, soaps, shampoos, foams, characterised in that it contains at least one of the compounds as defined in any one of Claims 1 to 5, in a physiologically acceptable vehicle, at a concentration of between 0.01 and 5 % by weight.

**19.** Composition according to Claim 18, characterised in that it contains, in addition, hydrating agents, antiseborrhoeic agents and agents promoting hair regrowth, steroidal or non-steroidal anti-inflammatory agents, carotenoids, and 5,8,11,14-eicosatetraynoic and 5,8,11-eicosatriynoic acids and their esters and amides.

**20.** Composition according to any one of Claims 15 to 19, characterised in that it also contains preservatives, stabilising agents and pH regulators, osmotic pressure modifying agents, emulsifying agents, UV-A and UV-B screening agents and antioxidants.

**21.** Process for cosmetic treatment of the hair, characterised in that at least one composition as defined in any one of Claims 15, 16 and 18 to 20 is applied to the scalp.

**22.** Use of the compounds according to any one of Claims 1 to 5, for the preparation of a medicinal product for the treatment of pelade, hair loss and desquamating dermatitis.

**Claims for the following Contracting State : ES**

**1.** Process for preparing the compound which corresponds to the general formula (I) or its isomeric form of formula (II):

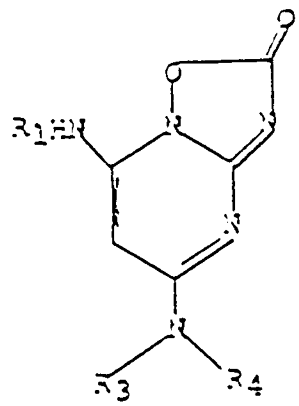
(I)

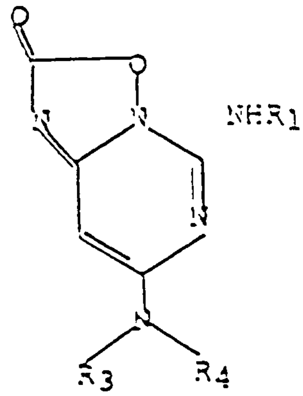
(II)

in which:

$R_3$ and $R_4$, which may be identical or different, denote a hydrogen atom, a linear or branched $C_1$-$C_{18}$ alkyl radical, an alkenyl radical having 2 to 18 carbon atoms or a cycloalkyl radical having 5 to 8 carbon atoms, these radicals optionally being substituted with one or more $C_1$-$C_6$ alkyl groups, it being possible for the alkyl, alkenyl or cycloalkyl groups themselves to be substituted with one or more hydroxyl group(s);

$R_3$ or $R_4$ also denotes an aryl and aralkyl group corresponding to the formula (III):

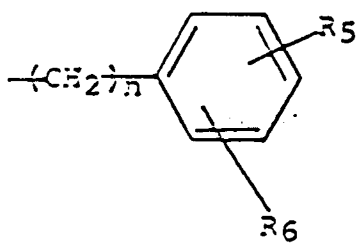
(III)

in which:

n can take values from 0 to 4, where $R_5$ and $R_6$, independently of one another, denote a hydrogen, a $C_1$-$C_6$ alkyl group, a nitro, hydroxyl or alkoxy group, or a halogen atom, alternatively a carboxyl group as well as its salified ester and amide forms;

$R_3$ and $R_4$, taken together with the nitrogen atom to which they are attached, can form a heterocycle chosen from morpholino, piperidino, pyrrolidino and piperazino groups and a 4'-(N-alkyl)-piperazino group in which the alkyl group at position 4' preferably contains 1 to 6 carbon atoms, one of which is optionally substituted with a hydroxyl group;

$R_1$ denotes a hydrogen atom or a carbamoyl group of formula:

$$-\underset{\underset{O}{\|}}{C}-NH-R_2$$

in which $R_2$ denotes a hydrogen atom, a linear or branched $C_1$-$C_{18}$ alkyl radical, a $C_2$-$C_{18}$ alkenyl group, a $C_5$-$C_8$ cycloalkyl group or an aryl or aralkyl radical corresponding to the formula (III) above, characterised in that:

(A) to prepare the compounds corresponding to the formula (Ia) or its isomeric form of formula (IIa):

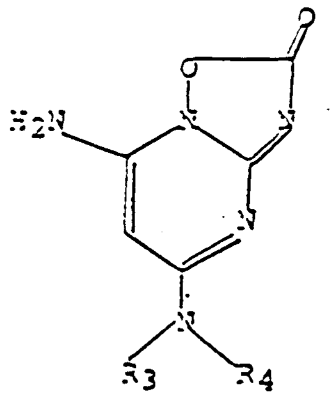

(Ia)

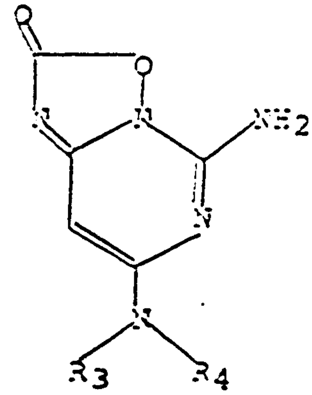

(IIa)

in which $R_3$ and $R_4$ have the meaning stated above, the monourea of the 2,4,6-triaminopyrimidine 3-oxide of formula (VIIa) or its isomeric form of formula (VIIb):

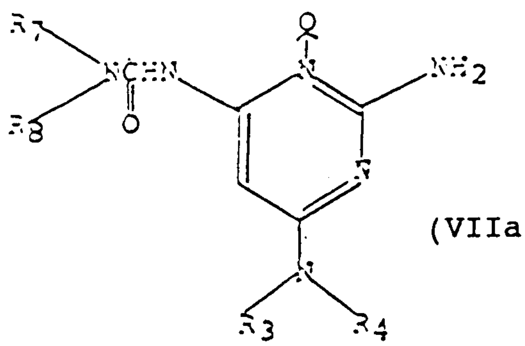

(VIIa)

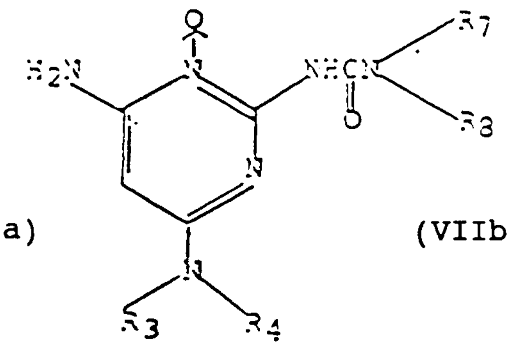

(VIIb)

in which $R_3$ and $R_4$ have the same meaning as that stated above and $R_7$ and $R_8$, independently of one another, denote a linear or branched $C_1$-$C_{18}$ alkyl radical, a $C_2$-$C_{18}$ alkenyl group, a $C_5$-$C_8$ cycloalkyl group or an aryl or aralkyl group corresponding to the formula (III) defined above, is treated by an elimination/cyclisation reaction in the presence of an organic solvent, bringing the monourea of formula (VIIb) to a temperature of between 40° and 100°C to obtain the compound of formula (Ia), or the monourea (VIIA) to a temperature above 100°C for a few hours to obtain the compound of formula (IIa);

(B) to prepare the compounds corresponding to formula (Ib) or its isomeric form of formula (IIb):

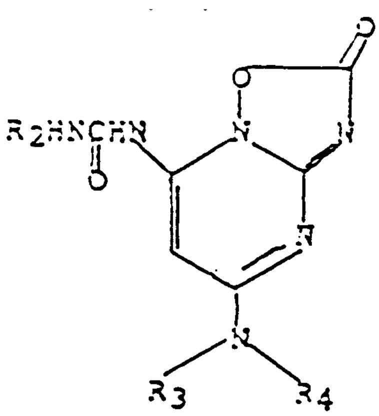

(Ib)

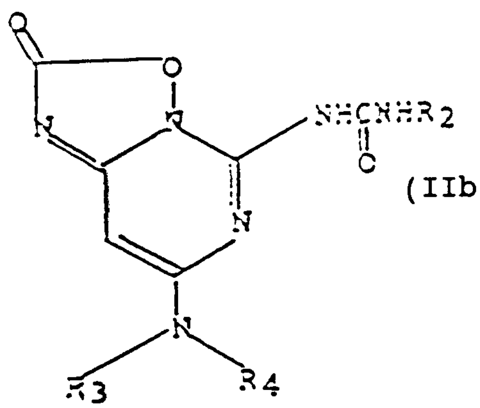

(IIb)

the urea of formula (VIIa) or (VIIb) is treated with an isocyanate of formula (IX):

$R_2$-N=C=O (IX)

in which $R_2$ has the meaning stated above, in dimethyl suphoxide (DMSO);

(C) to prepare the compound corresponding to formula (Ib) or (IIb) as defined above, the oxadiazolopyrimidinone of formula (Ia) or its isomer of formula (IIa):

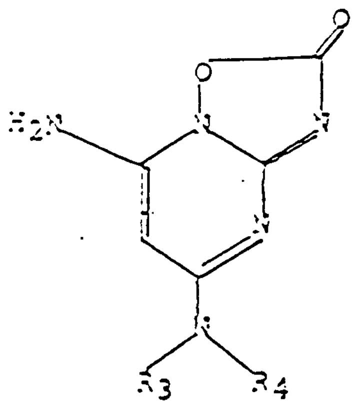

(Ia)

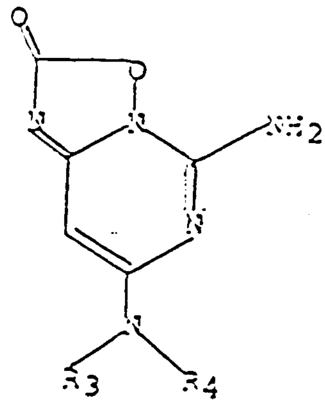

(IIa)

in which $R_3$ and $R_4$ have the same meaning as that stated above, is treated directly with the isocyanate of formula (IX) defined above, in an aprotic polar solvent;

(D) to prepare the compounds corresponding to the formula (Ib) defined above, a monourea of formula (Xa):

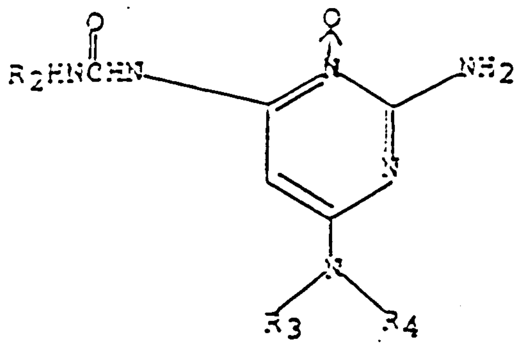

(Xa)

in which $R_2$, $R_3$ and $R_4$ have the same meaning as stated above, is treated in dioxane with a carbamoyl chloride of formula (V):

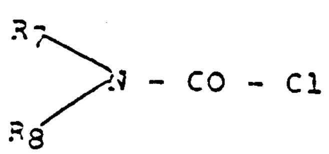

(V)

in which $R_7$ and $R_8$ have the meaning stated above, to obtain the diurea of formula (XIa):

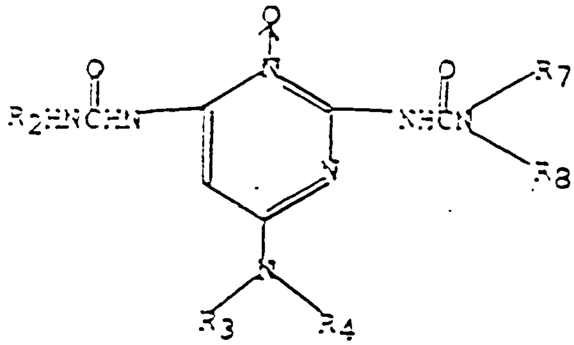

(XIa)

in which $R_2$, $R_3$, $R_4$, $R_7$ and $R_8$ have the same meaning as stated above, which is then brought to a temperature of between 50 and 120 ° C for a few hours in the presence of an organic solvent.

**2.** Preparation process according to Claim 1(A), characterized in that the compounds of formula (VIIa) and their isomers of formula (VIIb) are prepared by treating the 2,4,6-triaminopyrimidine 3-oxide of formula (IV):

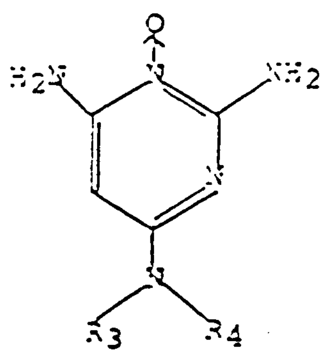

(IV)

in which $R_3$ and $R_4$ have the meaning stated in Claim 1, with a carbamoyl chloride of formula (V):

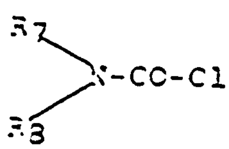

(V)

in which $R_7$ and $R_8$ have the meaning stated in Claim 1, with a polar aprotic solvent, to obtain the chloride of the following formula (VI):

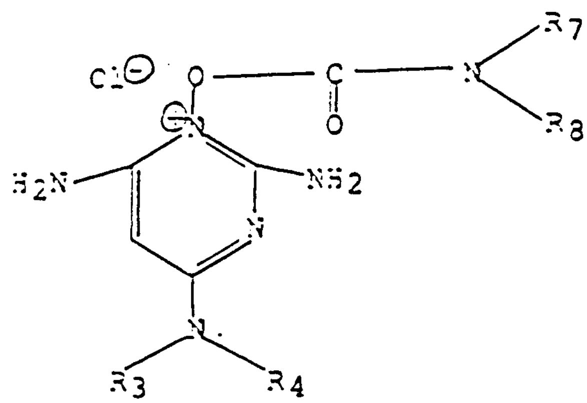

(VI)

which is then treated with one equivalent of strong base with respect to the carbamoyl chloride used.

**3.** Process according to Claim 1, characterised in that, in the formula (I) or the formula (II), the $C_1$-$C_{18}$ alkyl radical is chosen from methyl, ethyl, propyl, 2-ethylhexyl, octyl, dodecyl, hexadecyl and octadecyl radicals, in that the lower alkyl radical is chosen from methyl, ethyl, isopropyl, butyl and tert-butyl groups, in that the $C_2$-$C_{18}$ alkenyl groups are chosen from allyl, butenyl, hexenyl, dodecenyl, hexadecenyl and octadecenyl groups and in that the aryl or aralkyl groups are chosen from phenyl, 4-tolyl, 2-nitrophenyl, 4-nitrophenyl, 4-fluorophenyl, 4-chlorophenyl, 2-carboxyphenyl, 4-carboxyphenyl, 4-hydroxyphenyl, benzyl and phenethyl radicals.

**4.** Composition in the form of ointments, tinctures, creams, pomades, powders, patches, impregnated pads, solutions, emulsions, lotions, gels, sprays or anhydrous or aqueous suspensions, characterised in that it contains at least one compound obtained by the process of any of Claims 1 to 3, in a vehicle suitable for topical application, at a concentration of between 0.1 and 10 % by weight relative to the total weight of the composition, especially between 0.2 and 5 % by weight.

**5.** Cosmetic composition in the form of lotions, gels, soaps, shampoos, foams, characterised in that it contains at least one of the compounds such as that obtained by the process of any one of Claims 1 to 3, in a vehicle suitable for topical application, at a concentration of between 0.1 and 5 % by weight.

**6.** Composition according to Claim 5, characterised in that it contains, in addition, hydrating agents, antiseborrhoeic agents and agents promoting hair regrowth, steroidal or non-steroidal anti-inflammatory agents, carotenoids, and 5,8,11,14-eicosatetraynoic and 5,8,11-eicosatriynoic acids and their esters and amides.

**7.** Composition according to any one of Claims 4 to 6, characterised in that it also contains preservatives, stabilizing agents and pH regulators, osmotic pressure modifying agents, emulsifying agents, UV-A and UV-B screening agents and antioxidants.

**8.** Process for cosmetic treatment of the hair, characterised in that at least one composition as defined in any one of Claims 5 to 7 is applied to the scalp.

**Patentansprüche**
**Patentansprüche für folgende Vertragsstaaten : AT, BE, CH, DE, FR, GB, IT, LI, NL, SE**

**1.** Verbindung gemäß der allgemeinen Formel (I) oder ihrer isomeren Form der Formel (II):

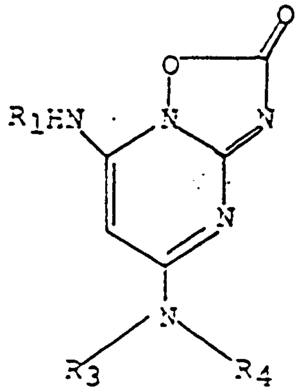 (I)

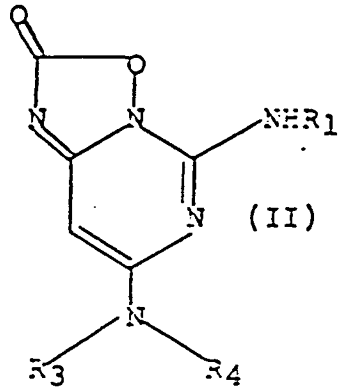 (II)

worin:

$R_3$ und $R_4$, gleich oder verschieden, ein Wasserstoffatom, einen linearen oder verzweigten $C_1$-$C_{18}$-Alkylrest, eine Alkenylgruppe mit 2 bis 18 Kohlenstoffatomen, eine Cycloalkylgruppe mit 5 bis 8 Kohlenstoffatomen, gegebenenfalls substituiert durch eine oder mehrere Niedrigalkylgruppen, darstellen, wobei die Alkyl-, Alkenyl- oder Cycloalkylgruppen ihrerseits durch eine oder mehrere Hydroxylgruppen substituiert sein können,

$R_3$ oder $R_4$ auch eine Aryl- oder Aralkylgruppe der Formel (III) darstellen:

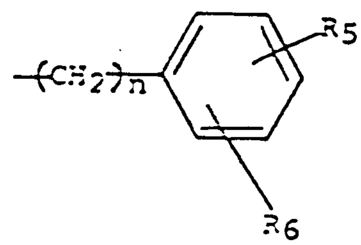 (III)

worin:

n eine ganze Zahl von 0 bis 4 ist, worin $R_5$ und $R_6$, unabhängig voneinander, Wasserstoff, eine $C_1$-$C_6$-Niedrigalkylgruppe, eine Nitro-, Hydroxyl-, Alkoxygruppe oder ein Halogenatom oder auch eine Carboxylgruppe sowie ihre Salz-, Ester- und Amidformen darstellen,

$R_3$ und $R_4$ zusammen mit dem Stickstoffatom, an das sie gebunden sind, einen Heterozyklus, ausgewählt aus Morpholino-, Piperidino-, Pyrrolidino-, Piperazino-, 4'-N-Alkylpiperazinogruppen, worin die Alkylgruppe in 4'-Position vorzugsweise 1 bis 6 Kohlenstoffatome enthält, wobei eines gegebenenfalls mit einer Hydroxylgruppe substituiert ist;

$R_1$ ein Wasserstoffatom oder eine Carbamoylgruppe der Formel darstellt:

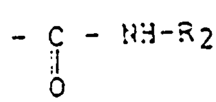

worin $R_2$ ein Wasserstoffatom, einen linearen oder verzweigten $C_1$-$C_{18}$-Alkylrest, eine $C_2$-$C_{18}$-Alkenylgruppe, eine $C_5$-$C_8$-Cycloalkylgruppe, einen Aryl- oder Aralkylrest der obigen Formel (III) darstellt, mit der Maßgabe, daß, wenn $R_1$ Wasserstoff darstellt, $R_3$ und $R_4$ zusammen mit dem Stickstoff, an den sie gebunden sind, nicht die [3,6-Dihydro-1(2H)-pyridyl]-Gruppe darstellen.

**2.** Verbindung gemäß Anspruch 1, dadurch gekennzeichnet, daß in Formel (I) oder Formel (II) der $C_1$-$C_{18}$-Alkylrest ausgewählt ist aus Methyl-, Ethyl-, Propyl-, Ethyl-2-hexyl-, Octyl-, Dodecyl-, Hexadecyl- und Octadecylresten, daß der Niedrigalkylrest ausgewählt ist aus Methyl-, Ethyl-, Isopropyl-, Butyl- und t-Butylgruppen, daß die $C_2$-$C_{18}$-Alkenylgruppen ausgewählt sind aus Allyl-, Butyl-, Hexenyl-, Dodecenyl-, Hexadecenyl- und Octadecenylgruppen, und daß die Aryl- oder Aralkylgruppen ausgewählt sind aus

Phenyl-, 4-Tolyl-, 2-Nitrophenyl-, 4-Nitrophenyl-, 4-Fluorphenyl-, 4-Chlorphenyl-, 2-Carboxyphenyl-, 4-Carboxyphenyl-, 4-Hydroxyphenyl-, Benzyl- und Phenethylresten.

**3.** Verbindung gemäß Anspruch 1 oder 2, dadurch gekennzeichnet, daß sie durch die Formel (Ia) oder ihre isomere Form der Formel (IIa) dargestellt ist:

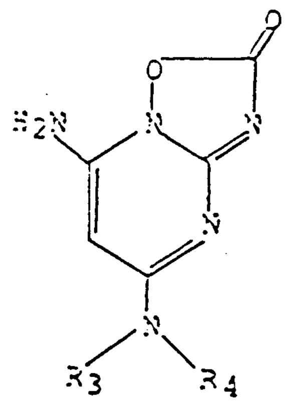

(Ia)

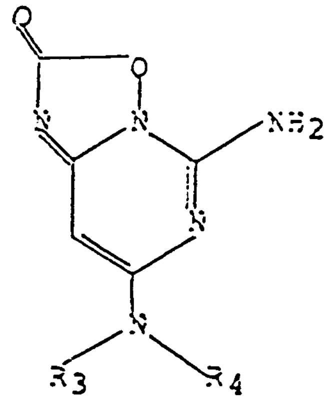

(IIa)

worin $R_3$ und $R_4$ die in Anspruch 1 angegebene Bedeutung haben.

**4.** Verbindung gemäß Anspruch 1 oder 2, dadurch gekennzeichnet, daß die Verbindung durch die Formel (Ib) oder ihre isomere Form (IIb) dargestellt ist:

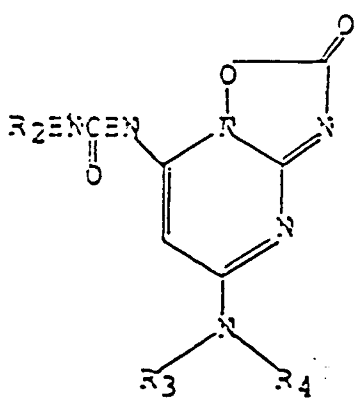

(Ib)

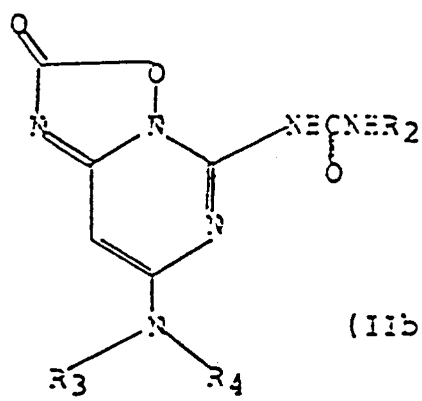

(IIb)

worin $R_2$, $R_3$ und $R_4$ die in Anspruch 1 angegebene Bedeutung haben.

**5.** Verbindung gemäß Anspruch 4, dadurch gekennzeichnet, daß sie durch die Formel (Ic) oder ihre isomere Form (IIc) dargestellt ist:

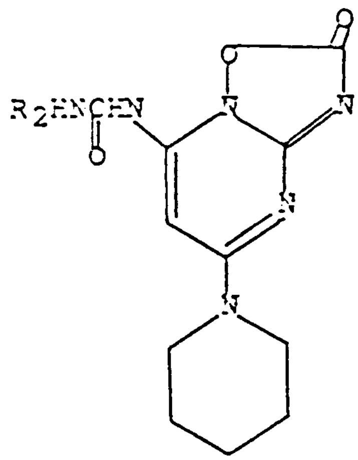
(Ic)

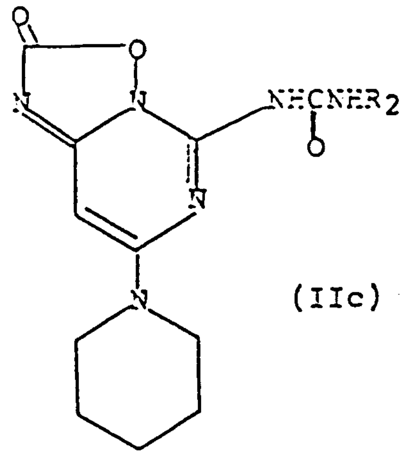
(IIc)

worin $R_2$ die in Ansprpuch 1 angegebene Bedeutung hat.

6. Verbindung, dadurch gekennzeichnet, daß sie durch die Formel (VIIa) oder ihre isomere Form (VIIb) dargestellt ist:

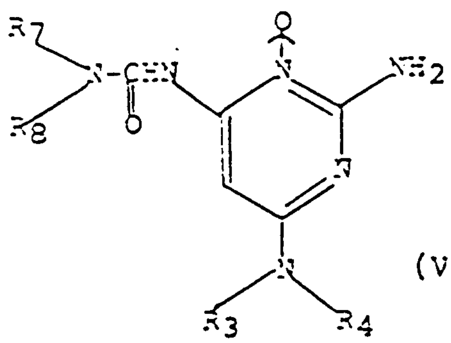
(VIIa)

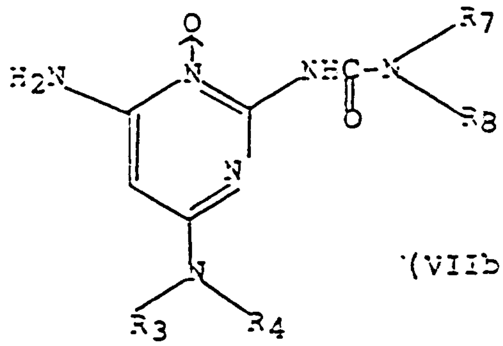
(VIIb)

worin $R_3$ und $R_4$ die in Anspruch 1 angegebene Bedeutung haben, und $R_7$ und $R_8$, unabhängig voneinander, einen linearen oder verzweigten $C_1$-$C_{18}$-Alkylrest, eine $C_2$-$C_{18}$-Alkenylgruppe, eine $C_5$-$C_8$-Cycloalkylgruppe, einen Aryl- oder Aralkylgruppe der in Anspruch 1 definierten Formel (III) darstellen.

7. Verfahren zur Herstellung der Verbindung gemäß Anspruch 1 oder 2, insbesondere der Verbindungen der Formel (Ia) oder (IIa):

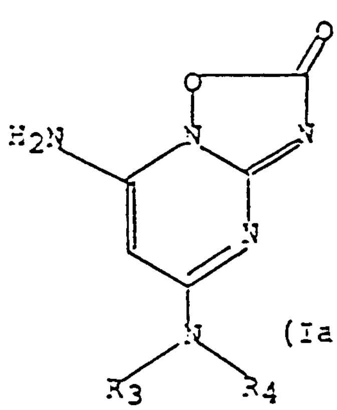
(Ia)

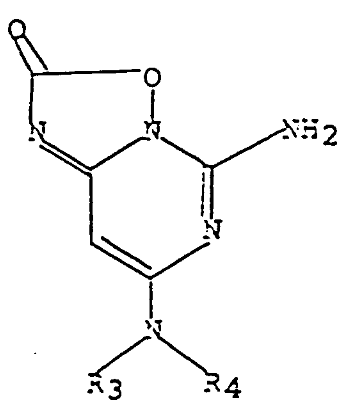
(IIa)

dadurch gekennzeichnet, daß man den Monoharnstoff des 2,4,6-Triaminopyrimidin-3-oxids der Formel

(VIIa) oder ihrer isomeren Form der Formel (VIIb):

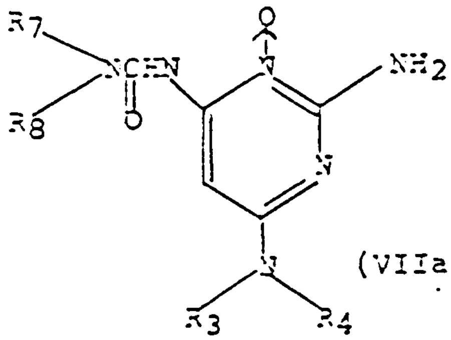

(VIIa)

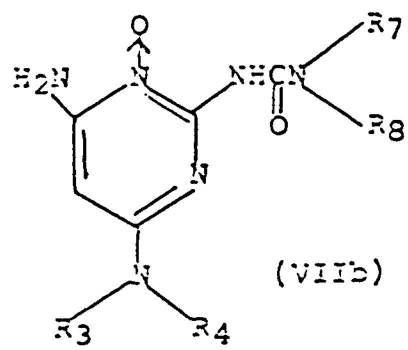

(VIIb)

worin $R_3$, $R_4$, $R_7$ und $R_8$ die in Anspruch 6 angegebene Bedeutung haben, einer Eliminierungs-Zyklisierungs-Reaktion in Gegenwart eines organischen Lösungsmittels unterzieht, wobei man den Monoharnstoff (VIIb) bei einer Temperatur von 40 bis 100 °C hält, um die Verbindung der Formel (Ia) zu erhalten, oder den Monoharnstoff (VIIa) bei einer Temperatur oberhalb 100 °C einige Stunden lang hält, um die Verbindung der Formel (IIa) zu erhalten.

**8.** Verbindung, dadurch gekennzeichnet, daß sie durch die Formel (VI) dargestellt ist:

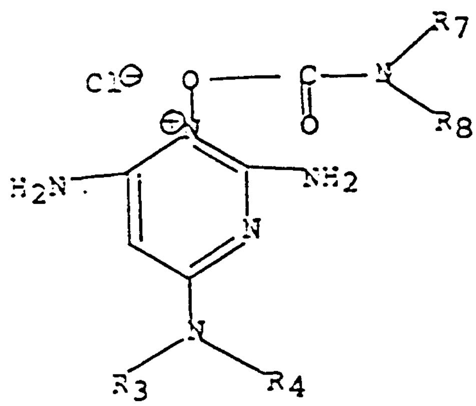

(VI)

worin $R_3$, $R_4$, $R_7$ und $R_8$ die in Anspruch 6 angegebene Bedeutung haben.

**9.** Herstellungsverfahren gemäß Anspruch 7, dadurch gekennzeichnet, daß die Verbindungen der Formel (VIIa) und ihre Isomeren der Formel (VIIb) hergestellt werden, indem man das 2,4,6-Triaminopyrimidin-3-oxid der Formel (IV):

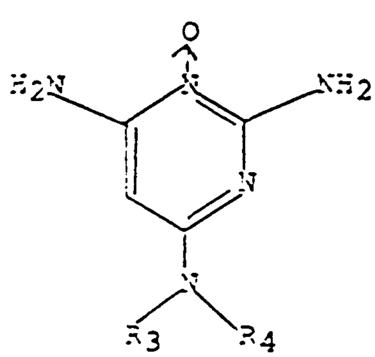

(IV)

worin $R_3$ und $R_4$ die in Anspruch 1 angegebene Bedeutung haben, mit einem Carbamoylchlorid der

Formel (V):

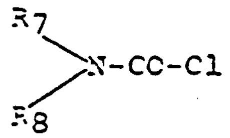

(V)

worin $R_7$ und $R_8$ die in Anspruch 6 angegebene Bedeutung haben, in einem polaren aprotischen Lösungsmittel behandelt, um das Chlorid der folgenden Formel (VI) zu erhalten:

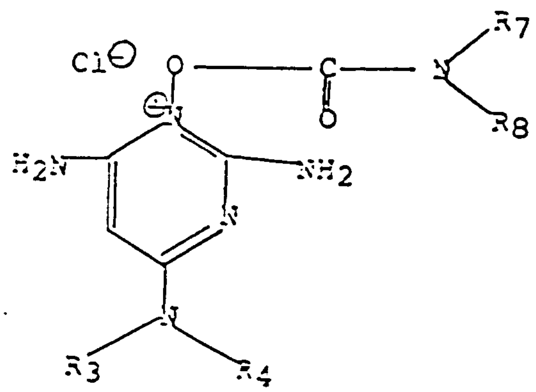

(VI)

welches man anschließend mit 1 Äquivalent starker Base, bezogen auf eingesetztes Carbamoylchlorid, behandelt.

**10.** Verfahren zur Herstellung der Verbindungen gemäß Anspruch 4, die durch Formel (Ib) oder ihre isomere Form (IIb) dargestellt sind:

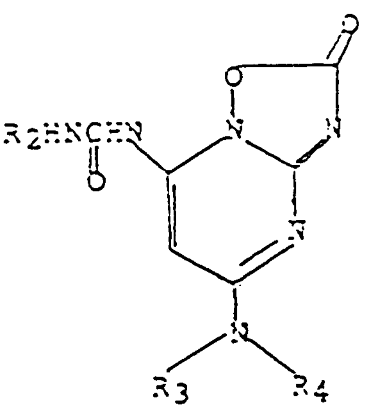

(Ib)

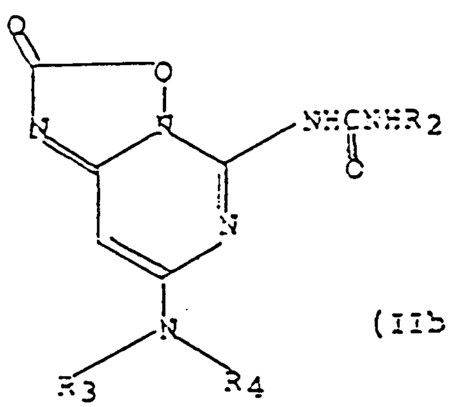

(IIb)

dadurch gekennzeichnet, daß man den Harnstoff der Formel (VIIa) oder (VIIb) mit einem Isocyanat der Formel (IX) in Dimethylsulfoxid (DMSO) behandelt:

$R_2\text{-}N=C=O$ (IX)

worin $R_2$ die in Anspruch 1 angegebene Bedeutung hat.

**11.** Verfahren zur Herstellung der Verbindungen gemäß Anspruch 4, die durch die Formel (Ib) oder (IIb) dargestellt sind, dadurch gekennzeichnet, daß man direkt das Oxadiazolopyrimidinon der Formel (Ia) oder sein Isomer der Formel (IIa):

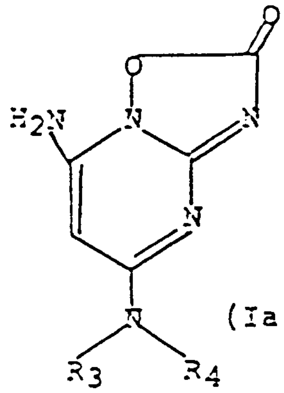
(Ia)

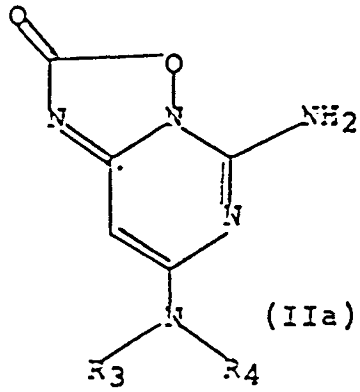
(IIa)

mit dem Isocyanat der Formel (IX) in einem polaren aprotischen Lösungmittel behandelt.

**12.** Verfahren zur Herstellung der Verbindung der Formel (Ib) gemäß Anspruch 4, dadurch gekennzeichnet, daß man einen Monoharnstoff der Formel (Xa):

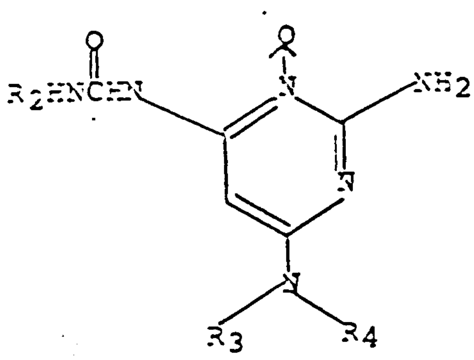
(Xa)

worin $R_2$, $R_3$ und $R_4$ die in Anspruch 1 angegebene Bedeutung haben, in Dioxan mit einem Carbamoylchlorid der Formel (V), wie in Anspruch 9 definiert, behandelt, um den Diharnstoff der Formel (XIa) zu erhalten:

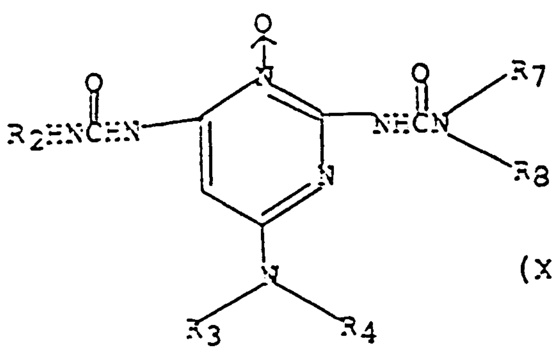
(XIa)

welchen man anschließend bei einer Temperatur von 50 bis 120°C einige Stunden lang in der Gegenwart eines organischen Lösungsmittels hält.

**13.** Verbindung, dadurch gekennzeichnet, daß sie durch die Formel (VIIIa) dargestellt ist:

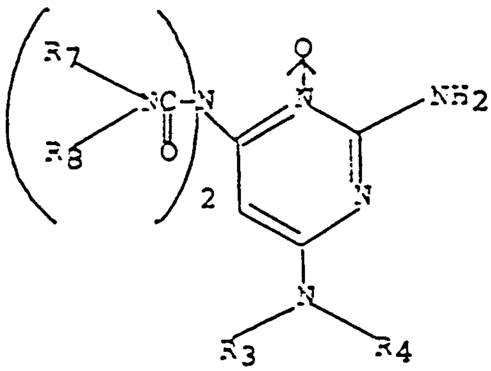

(VIIIa)

worin $R_3$, $R_4$, $R_7$ und $R_8$ die in Anspruch 6 angegebenen Bedeutungen haben.

**14.** Verbindung, dadurch gekennzeichnet, daß sie durch die Formel (XIa) dargestellt ist:

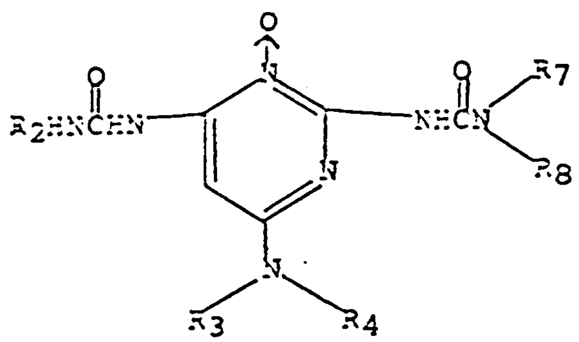

(XIa)

worin $R_2$, $R_3$, $R_4$, $R_7$, $R_8$ die in den Ansprüchen 1 bis 6 definierten Bedeutungen haben.

**15.** Kosmetische oder pharmazeutische Zusammensetzung, dadurch gekennzeichnet, daß sie in einem zur topischen Aufbringung geeigneten Träger mindestens eine Verbindung gemäß eines jeden der Ansprüche 1 bis 5 enthält.

**16.** Zusammensetzung gemäß Anspruch 15, dadurch gekennzeichnet, daß sie in Form von Salben, Tinkturen, Cremes, Pomaden, Pulver, Puder, getränkten Tampons, Lösungen, Emulsionen, Lotionen, Gelen, Sprays oder wasserfreien oder wäßrigen Suspensionen vorliegt.

**17.** Pharmazeutische Zusammensetzung gemäß Anspruch 15, dadurch gekennzeichnet, daß die Verbindung der Formel (I) in Konzentrationen von 0,1 bis 10 Gew.-%, vorzugsweise von 0,2 bis 5 Gew.-%, bezogen auf Gesamtgewicht der Zusammensetzung, vorliegt.

**18.** Kosmetische Zusammensetzung gemäß Anspruch 15, in Form von Lotionen, Gelen, Seifen, Shampoos, Schäumen, dadurch gekennzeichnet, daß sie in einem physiologisch verträglichen Träger mindestens eine der Verbindungen gemäß eines jeden der Ansprüche 1 bis 5 in einer Konzentration von 0,01 bis 5 Gew.-% enthält.

**19.** Zusammensetzung gemäß Anspruch 18, dadurch gekennzeichnet, daß sie zusätzlich Hydratisiermittel, antiseborreische Mittel und Mittel zur Begünstigung des Haarwachstums, entzündungshemmende Mittel auf Basis von steroiden Dienen oder nicht-steroiden Dienen, Carotinoide, Eicosatetrainsäure-5,8,11,14 und Eicosatriinsäure-5,8,11 und deren Ester und Amide enthält.

**20.** Zusammensetzung gemäß eines jeden der Ansprüche 15 bis 19, dadurch gekennzeichnet, daß sie auch Konservierungsmittel, Stabilisierungsmittel, pH-Regulierungsmittel, Mittel zur Modifizierung des osmotischen Druckes, Emulgiermittel, UV-A- und UV-B-Filtermittel und Antioxidantien enthält.

**21.** Verfahren zu kosmetischen Behandlung der Haare, dadurch gekennzeichnet, daß man auf die behaarte Haut mindestens eine Zusammensetzung gemäß eines jeden der Ansprüche 15, 16 und 18 bis 20 aufträgt.

**22.** Verwendung der Verbindungen gemäß eines jeden der Ansprüche 1 bis 5 zur Herstellung eines Medikaments zur Behandlung von Pelade, Haarausfall und schuppender Dermatitis.

**Patentansprüche für folgenden Vertragsstaat : ES**

**1.** Verfahren zur Herstellung der Verbindung, die durch die allgemeine Formel (I) oder ihre isomere Form der Formel (II) dargestellt ist:

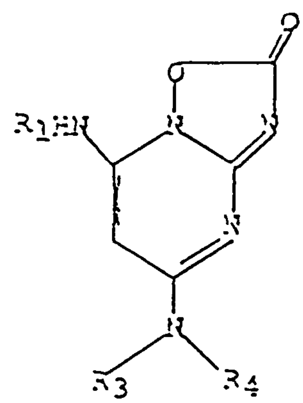

(I)

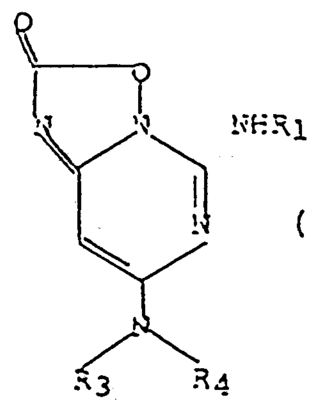

(II)

worin:

$R_3$ und $R_4$, gleich oder verschieden, ein Wasserstoffatom, einen linearen oder verzweigten $C_1$-$C_{18}$-Alkylrest, eine Alkenylgruppe mit 2 bis 18 Kohlenstoffatomen, eine Cycloalkylgruppe mit 5 bis 8 Kohlenstoffatomen, gegebenenfalls substituiert durch eine oder mehrere Niedrigalkylgruppen, darstellen, wobei die Alkyl-, Alkenyl- oder Cycloalkylgruppen ihrerseits durch eine oder mehrere Hydroxylgruppen substituiert sein können,

$R_3$ oder $R_4$ auch eine Aryl- oder Aralkylgruppe der Formel (III) darstellen:

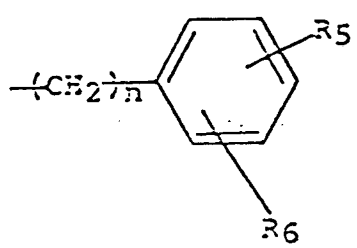

(III)

worin:

n eine ganze Zahl von 0 bis 4 ist, worin $R_5$ und $R_6$, unabhängig voneinander, Wasserstoff, eine $C_1$-$C_6$-Niedrigalkylgruppe, eine Nitro-, Hydroxyl-, Alkoxygruppe oder ein Halogenatom oder auch eine Carboxylgruppe sowie ihre Salz-, Ester- und Amidformen darstellen,

$R_3$ und $R_4$ zusammen mit dem Stickstoffatom, an das sie gebunden sind, einen Heterozyklus, ausgewählt aus Morpholino-, Piperidino-, Pyrrolidino-, Piperazino-, 4'-N-Alkylpiperazinogruppen, worin die Alkylgruppe in 4'-Position vorzugsweise 1 bis 6 Kohlenstoffatome enthält, wobei eines gegebenenfalls mit einer Hydroxylgruppe substituiert ist;

$R_1$ ein Wasserstoffatom oder eine Carbamoylgruppe der Formel darstellt:

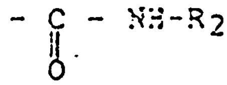

worin $R_2$ ein Wasserstoffatom, einen linearen oder verzweigten $C_1$-$C_{18}$-Alkylrest, eine $C_2$-$C_{18}$-Alkenylgruppe, eine $C_5$-$C_8$-Cycloalkylgruppe, einen Aryl- oder Aralkylrest der obigen Formel (III) darstellt, dadurch gekennzeichnet, daß:

(A) zur Herstellung der Verbindungen der Formel (Ia) oder ihrer isomeren Form der Formel (IIa):

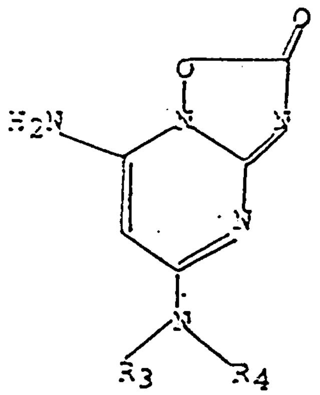
(Ia)

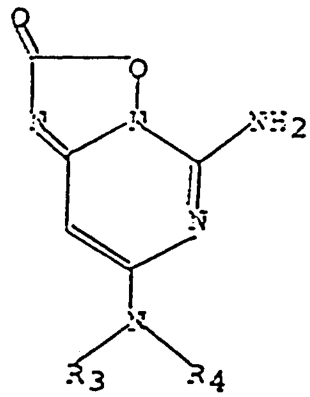
(IIa)

worin $R_3$ und $R_4$ die oben angegebene Bedeutung haben, man den Monoharnstoff des 2,4,6-Triaminopyrimidin-3-oxids der Formel (VIIa) oder ihrer isomeren Form der Formel (VIIb):

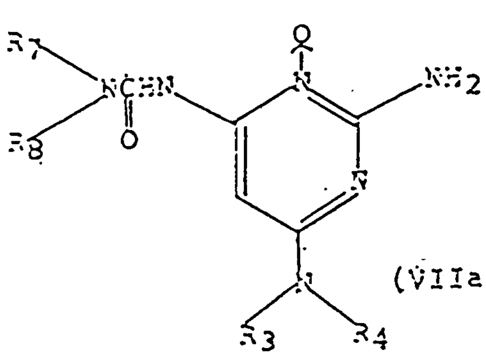
(VIIa)

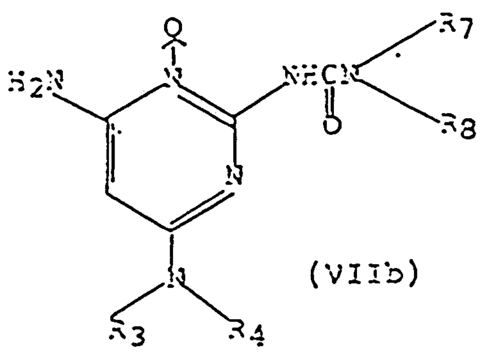
(VIIb)

worin $R_3$ und $R_4$ dieselbe Bedeutung wie oben angegeben haben, und $R_7$ und $R_8$, unabhängig voneinander, einen linearen oder verzweigten $C_1$-$C_{18}$-Alkylrest, eine $C_2$-$C_{18}$-Alkenylgruppe, eine $C_5$-$C_8$-Cycloalkylgruppe, eine Aryl- oder Aralkylgruppe der oben definierten Formel (III) darstellen, einer Eliminierungs-Zyklisierungs-Reaktion in Gegenwart eines organischen Lösungsmittel unterzieht, wobei man den Monoharnstoff der Formel (VIIb) bei einer Temperatur von 40 bis 100 °C hält, um die Verbindung der Formel (Ia) zu erhalten, oder den Monoharnstoff (VIIa) bei einer Temperatur oberhalb 100 °C einige Stunden lang hält, um die Verbindung der Formel (IIa) zu erhalten;

(B) zur Herstellung der Verbindungen gemäß Formel (Ib) oder ihrer isomeren Form der Formel (IIb):

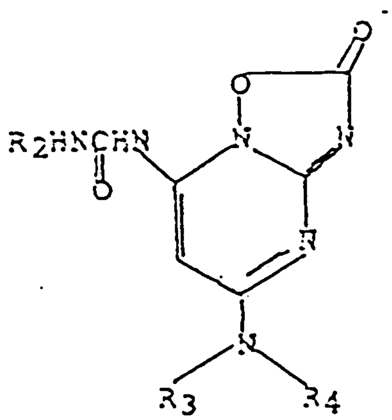

(Ib)

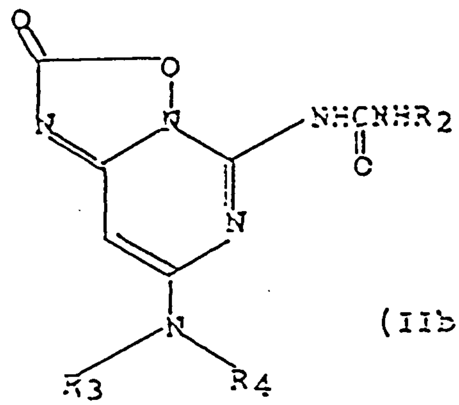

(IIb)

man den Harnstoff der Formel (VIIa) oder (VIIb) mit einem Isocyanat der Formel (IX) in Dimethylsulfoxid (DMSO) behandelt:

$R_2$-N = C = O (IX)

worin $R_2$ die in Anspruch 1 angegebene Bedeutung hat;

(C) zur Herstellung der Verbindung der Formel (Ib) oder (IIb), wie oben definiert, man direkt das Oxidiazolopyrimidinon der Formel (Ia) oder ihr Isomer der Formel (IIa):

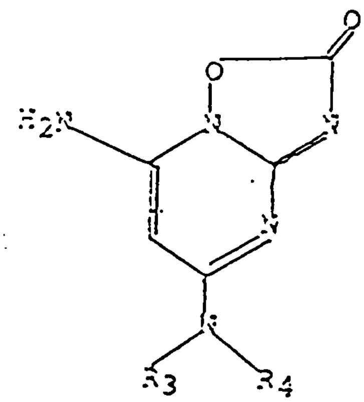

(Ia)

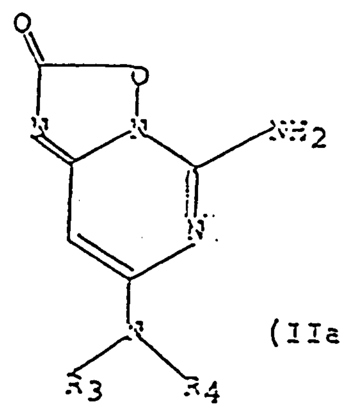

(IIa)

worin $R_3$ und $R_4$ dieselbe Bedeutung wie oben haben, mit einem Isocyanat der oben definierten Formel (IX) in einem polaren aprotischen Lösungsmittel behandelt;

(D) zur Herstellung der Verbindungen gemäß der oben definierten Formel (Ib) man einen Monoharnstoff der Formel (Xa):

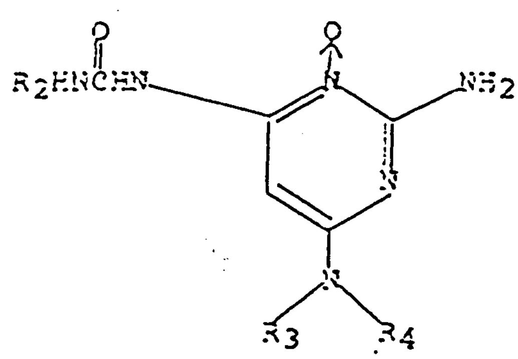

(Xa)

worin $R_2$, $R_3$ und $R_4$ die oben angegebene Bedeutung haben, in Dioxan mit einem Carbamoylchlorid der Formel (V) behandelt:

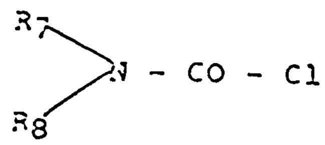

(V)

worin $R_7$ und $R_8$ die oben angegebene Bedeutung haben, um den Diharnstoff der Formel (XIa) zu erhalten:

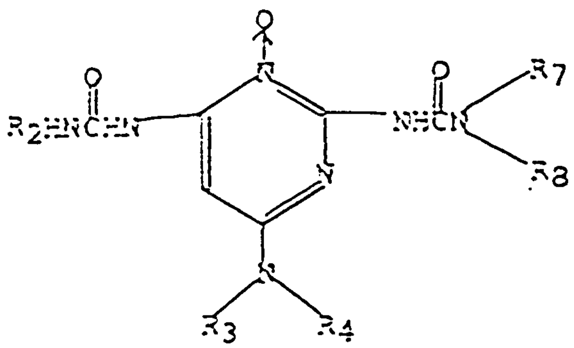

(XIa)

worin $R_2$, $R_3$, $R_4$, $R_7$ und $R_8$ die oben angegebene Bedeutung haben, welchen man anschließend bei einer Temperatur von 50 bis 120 °C einige Stunden lang in der Gegenwart eines organischen Lösungsmittels hält.

2. Herstellungsverfahren gemäß Anspruch 1(A), dadurch gekennzeichnet, daß man die Verbindungen der Formel (VIIa) und ihre Isomeren der Formel (VIIb) herstellt, indem man das 2,4,6-Triaminopyrimidin-3-oxid der Formel (IV):

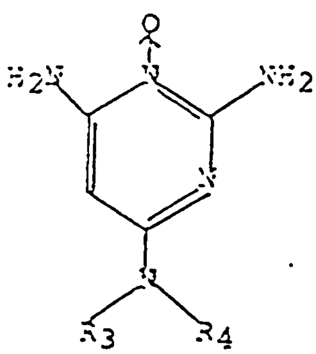

(IV)

worin $R_3$ und $R_4$ die in Anspruch 1 angegebene Bedeutung haben, mit einem Carbamoylchlorid der Formel (V) in einem aprotischen polaren Lösungsmittel behandelt:

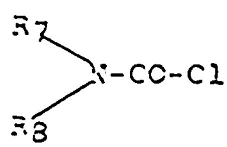

(V)

worin $R_7$ und $R_8$ die in Anspruch 1 angegebene Bedeutung haben, um das Chlorid der folgenden Formel (VI) zu erhalten:

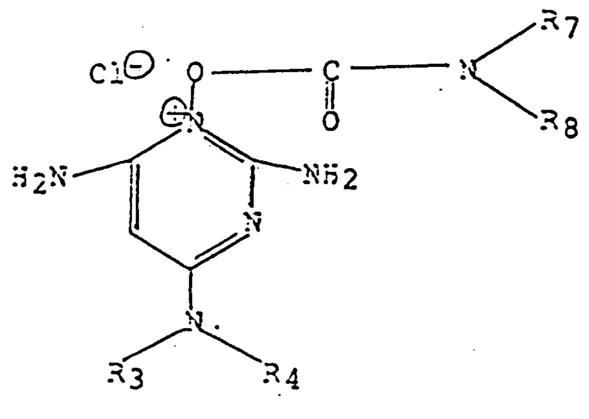

(VI)

welches man anschließend mit 1 Äquivalent starker Base, bezogen auf eingesetztes Carbamoylchlorid, behandelt.

3. Verfahren gemäß Anspruch 1, dadurch gekennzeichnet, daß in Formel (I) oder Formel (II) der $C_1$-$C_{18}$-Alkylrest ausgewählt ist aus Methyl-, Ethyl-, Propyl-, Ethyl-2-hexyl-, Octyl-, Dodecyl-, Hexadecyl- und Octadecylresten, daß der Niedrigalkylrest ausgewählt ist aus Methyl-, Ethyl-, Isopropyl-, Butyl- und t-Butylgruppen, daß die $C_2$-$C_{18}$-Alkenylgruppen ausgewählt sind aus Allyl-, Butyl-, Hexenyl-, Dodecenyl-, Hexadecenyl- und Octadecenylgruppen, und daß die Aryl- oder Aralkylgruppen ausgewählt sind aus Phenyl-, 4-Tolyl-, 2-Nitrophenyl-, 4-Nitrophenyl-, 4-Fluorphenyl-, 4-Chlorphenyl-, 2-Carboxyphenyl-, 4-Carboxyphenyl-, 4-Hydroxyphenyl-, Benzyl- und Phenethylresten.

4. Zusammensetzung in Form von Salben, Tinkturen, Cremes, Pomaden, Pulvern, Puder, getränkten Tampons, Lösungen, Emulsionen, Lotionen, Gelen, Sprays oder wasserfreien oder wäßrigen Suspensionen, dadurch gekennzeichnet, daß sie in einem zur topischen Aufbringung geeigneten Träger mindestens eine Verbindung, erhältlich durch das Verfahren gemäß eines jeden der Ansprüche 1 bis 3, in einer Konzentration von 0,1 bis 10 Gew.-%, vorzugsweise von 0,2 bis 5 Gew.-%, bezogen auf Gesamtgewicht der Zusammensetzung, enthält.

5. Kosmetische Zusammensetzung in Form von Lotionen, Gelen, Seifen, Shampoos, Schäumen, dadurch gekennzeichnet, daß sie in einem zur topischen Aufbringung geeigneten Träger mindestens eine der Verbindungen, erhältlich durch das Verfahren gemäß eines jeder der Ansprüche 1 bis 3, in einer Konzentration von 0,01 bis 5 Gew.-% enthält.

6. Zusammensetzung gemäß Anspruch 5, dadurch gekennzeichnet, daß sie zusätzlich Hydratisiermittel, antiseborreische Mittel und Mittel zur Begünstigung des Haarwachstums, entzündungshemmende Mittel auf Basis von steroiden Dienen oder nicht-steroiden Dienen, Carotinoide, Eicosatetrainsäure-5,8,11,14 und Eicosatriinsäure-5,8,11 und deren Ester und Amide enthält.

7. Zusammensetzung gemäß einem jeden der Ansprüche 4 bis 6, dadurch gekennzeichnet, daß sie auch Konservierungsmittel, Stabilisierungsmittel, pH-Regulierungsmittel, Mittel zur Modifizierung des osmotischen Druckes, Emulgiermittel, UV-A- und UV-B-Filtermittel und Antioxidantien enthält.

8. Verfahren zur kosmetischen Behandlung der Haare, dadurch gekennzeichnet, daß man auf die behaarte Haut mindestens eine Zusammensetzung gemäß eines jeden der Ansprüche 5 bis 7 aufträgt.